(19) **Europäisches Patentamt / European Patent Office / Office européen des brevets**

(11) **EP 4 437 858 A1**

(12) **EUROPEAN PATENT APPLICATION**

(43) Date of publication:
**02.10.2024 Bulletin 2024/40**

(21) Application number: **23382313.7**

(22) Date of filing: **31.03.2023**

(51) International Patent Classification (IPC):
**A23L 33/12** (2016.01)      **A23L 33/00** (2016.01)
**A61K 31/202** (2006.01)      **A61P 29/00** (2006.01)

(52) Cooperative Patent Classification (CPC):
**A23L 33/40; A23L 33/12; A61K 31/202;
A61P 29/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Fundación Para la Investigación del
Hospital
Universitario y Politécnico La Fe de la Comunidad
Valenciana
46026 Valencia (ES)**

(72) Inventors:
• **Kuligowski, Julia
46026 Valencia (ES)**

• **Sepúlveda Sanchis, Pilar
46026 Valencia (ES)**
• **Ten Doménech, Isabel
46026 Valencia (ES)**
• **Gómez Ferrer, Marta
46026 Valencia (ES)**
• **Albiach Delgado, Abel
46026 Valencia (ES)**
• **Vento Torres, Máximo
46026 Valencia (ES)**
• **Peiró Molina, Esteban
46026 Valencia (ES)**

(74) Representative: **De Arpe Tejero, Manuel
Arpe Patentes y Marcas
Edificio Aqua, Calle Agustín de Foxá 4-10
C.P. 28036 Madrid (ES)**

(54) **COMPOSITION COMPRISING OXYLIPINS PRESENT IN HUMAN MILK DERIVED SMALL
EXTRACELLULAR VESICLES AND ITS USE IN THE PREVENTION AND TREATMENT OF
INTESTINAL DISEASES**

(57) The present invention refers to a composition comprising a combination of oxylipins 14-HDHA, 17-HDHA, and 19,20-DiHDPA and, additionally, one or more oxylipins selected from the group consisting of 12,13-DiHOME; 9,10-DiHOME; 14,15-DiHETrE; $PGE_2$; $PGF_{2\alpha}$; 17,18-DiHETE; 11,12-DiHETrE; 9-HOTrE; 20-HETE; 12(S)-HpETE; 12(S)-HEPE; 13-HODE; 5(S)-HEPE; 9-HODE; 15-HETE; 16(17)-EpDPE; 13-HpODE; 13-KODE; 9-HpODE; 15-HpETE; 15-KETE; 11-HETE; 9-KODE; 12-HETE; 8-HETE; 15(S)-HETrE; 9-HETE; 5-HETE; 19(20)-EpDPE; 12(13)-EpOME; 5(S)-HpETE; 9(10)-EpOME; 12-KETE; 5-KETE; 8(9)-EpETrE, and 5(6)-EpETrE. The invention refers to the use of this composition as nutritional suplement. Finally, pharmaceutical compositions comprising this composition for the prevention and treatment of intestinal diseases are also contemplated.

EP 4 437 858 A1

**Description**

**Field of the invention**

The present invention relates generally to the Health Sector, and more particular to the prevention and treatment of intestinal diseases. Specifically, the invention relates to compositions comprising lipids, including oxylipins, present in human milk (HM) small extracellular vesicles (sEVs) to be used as nutritional supplements or pharmaceutical compositions for the prevention and treatment of necrotising enterocolitis (NEC) in preterm infants (PIs).

**Background of the invention**

In Spain, around 22,000 PIs are born each year, with an average hospitalization cost of €40,000 and an average stay of 50 days.

HM has several nutritional and immunological benefits that favour the clinical evolution and neurodevelopment of PIs in the short and long term. PIs fed with HM, especially own mother's milk (OMM), show a significant reduction, with respect to PIs fed with infant formula, in the incidence of serious diseases such as NEC, neonatal sepsis, bronchopulmonary dysplasia and retinopathy of prematurity, among others.

NEC is an acute bowel necrosis that mainly affect PIs, in which the mortality rate is 23.5%. Survivors may have serious sequelae, ranging from gastrointestinal complications, to severe neurodevelopmental retardation. Since the initial description of NEC, there has been little change in total mortality and treatment ourcomes. Thus, new strategies are needed to prevent this disease.

HM is a complex matrix with an overall composition of 87% water, 7% lactose, 3.8% fat and 1% protein. Among the components present in HM, Evs, which are present in high concentration, have been shown to be responsible for regulating intracellular communication, inflammation and immune response (Ascanius S. R. et al "Milk-Derived Extracellular Vesicles Suprress Inflammatory Cytorkine Expression and Nuclear Factor-κB Activation in Lipopolysaccharide-Stimulated Macrophages" Dairy 2021, 2, 165-178). These lipid bilayer membrane vesicles are able to encapsulate proteins, microRNAs, mRNAs and other biomolecules, protecting them from enzyme degradation. Lipid composition in HM and in HM-derived sEVs is different, since in the latter it is linked to the composition of the cell membrane due to their formation mechanisms, encompassing phospholipids, sphingolipids, and cholesterol. Recent advancements in high-resolution hyphenated liquid chromatography-mass spectrometry (LC-MS) have led to an increased interest in the understudied area of lipid analysis in Evs. In this context, the authors of the present invention have described the lipid content of HM-derived sEVs (Ramos-Garcia et al. ATR-FTIR spectroscopy for the routine quality control of exosome isolations. Chemometrics and Intelligent laboratory systems 2021). sEVs are defined by their size as nanovesicles ranging from < 100 nm to < 200 nm (Théry, C. et al. Minimal information for studies of extracellular vesicles 2018 (MISEV2018): a position statement of the International Society for Extracellular Vesicles and update of the MISEV2014 guidelines. J. Extracell. Vesicles 7, 1535750 (2018)). These type of vesicles are also called exosomes.

Regarding specific lipid families, HM contains two essential fatty acids, linoleic acid (LA) ($\omega$6) at 15% and alpha-linolenic acid ($\omega$3) at 0.35% of the total fatty acids present in HM. Elongation of these two essential fatty acids gives rise to arachidonic acid (AA, $\omega$6) and eicosapentaenoic acid (EPA, $\omega$3), respectively, and the latter to docosahexaenoic acid (DHA, $\omega$3). AA, EPA, and DHA are important for regulating growth, immune function, vision, cognitive development and motor systems in newborns. In particular, the synthesis of AA and DHA is limited in the foetus and neonate. Thus, the necessary amounts of AA and DHA must come from the mother during pregnancy, or through HM after birth. In preterm birth, the transmission of these fatty acids from the placenta is interrupted. Studies have shown that decreased postnatal blood levels of DHA and AA in Pis are associated with neonatal morbidities. Indeed, after birth, the PI is dependent on an adequate diet for sufficient levels of fatty acids. The addition of DHA and AA to PI formulas produced beneficial effects compared to Pis who did not receive supplementation (Martin, C. R. et al. (2011). Decreased postnatal docosahexaenoic and arachidonic acid blood levels in premature infants are associated with neonatal morbidities. The Journal of Pediatrics, 159(5), 743-749.e7492).

The intestinal epithelium is essentially a barrier that separates the organism from the bacteria present in the gastrointestinal tract. When this function is impaired, bacterial translocation from the intestines to the blood can occur, leading to infection and sepsis. The gastrointestinal tract of the PI is immature. After birth, nutrients from HM facilitate its maturation.

The contribution of sEVs to the maturation of the intestinal barrier has been studied in physiological and pathological models. Four main effects can be distinguished: (i) proliferation, (ii) integrity (cell-cell junctions), (iii) resolution of inflammation and (iv) mucin production (additional protection). WO202218889A1 proposes the use of HM sEVs or exosomes in the preparation of intestinal epithelial barrier protein protective agents or damage repair agents.

Furthermore, an *in vivo* study on a NEC animal model showed that the administration of exosomes isolated from HM reduced inflammation and promoted mucosal generation, thus preventing the development of NEC (Chen W.

et al "Lipidomic Profiling of Human Milk Derived Exosomes and Their Emerging Roles in the prevention of Necrotizing Enterocolitis". Mol Nutr. Food Res. 2021, 2000845).

However, at present, the mechanism of HM sEVs to prevent or treat neonatal NEC is unknown.

The study of the biochemical composition of HM sEVs is essential to understand their function. The protein and miRNA content of sEVs is currently being analysed, but little is known about the lipid composition of sEVs from HM (Buratta S. et al "Protein and Lipid Content of Milk Extracellular Vesicles: A comparative Overview" Life 2023, 13, 401).

Additionally, to date, despite the findings cited above, the isolation of sEVs from HM in large quantities for their commercialization (e.g. as a nutritional supplement for newborns) has not been addressed, mainly due to the need for donors, and also, the lack of an escalation procedure that allows such commercialization to be profitable.

In response to the needs of the state of the art, the authors of the present invention have carried out an important experimental work to characterize the lipid profiles of HM-derived sEVs, which has allowed the identification of certain oxylipins, including several lipid mediators, in HM-derived sEVs and differences in their concentrations compared to those found in HM.

Oxylipins are bioactive lipid mediators derived from long chain polyunsaturated fatty acids (LCPUFAs); LCPUFAs can be classified into two main categories: omega-3 (w3) and omega-6 (ω6). The intake of ω3 and ω6 LCPUFAs is associated with higher levels of ω3 and ω6 LCPUFA-derived oxylipins, respectively (Gila-Diaz, A., et al. (2021). Specialized Pro-Resolving Lipid Mediators in Neonatal Cardiovascular Physiology and Diseases. Antioxidants, 10(6), 933. Doi: 10.3390/antiox10060933. PMID: 34201378; PMCID: PMC8229722). AA, EPA, and DHA are the three main LCPUFAs that serve as precursors for the syhthesis of oxylipins. The latter play a role in various physiological processes, including inflammation, vascular homeostasis, and immune response. In contrast, specialized pro-resolving lipid mediators (SPMs), also derived from polyunsaturated fatty acids, have been found to promote the resolution of inflammation and tissue repair, leading to the restoration of homeostasis. While oxylipins can have pro-inflammatory or anti-inflammatory effects depending on the specific mediator and context, SPMs are primarily anti-inflammatory and pro-resolving in nature. Indeed, some oxylipins act as intermediate metabolites of SPM synthesis (Serhan CN, Levy BD. Resolvins in inflammation: emergence of the pro-resolving superfamily of mediators. J Clin Invest. 2018;128(7):2657-2669. Doi:10.1172/JCI97943). For example, the family of SPMs called resolvins are derived from EPA and DHA, while protectins and maresins are derived from DHA.

Author's research has shown that both, HM-derived sEVs and a panel of certain oxylipins, have a similar pro-resolving effect in several *in vitro* and *in vivo* experiments, from which it follows that the therapeutic effect of sEVs is mediated by different combinations of certain oxylipins.

The oxylipins prevalently found in HM are 9,10-DiHOME and 12,13-DiHOME (derived from LA, ω6), which tend to have pro-inflammatory effects. However, authors' analysis of the oxylipin profile in HM-derived sEVs showed that the relative concentration of these two oxylipins is lower and certain oxylipins, including 14-HDHA (14-hydroxydocosahexaenoic acid), 17-HDHA (17-hydroxydocosahexaenoic acid), and 19,20-DiHDPA (19,20-dihydroxydocosapentaenoic acid) (derived from DHA), for which anti-inflammatory activity has been described, are more prevalent.

The addition of HM-derived sEVs and these DHA-derived oxylipins (14-HDHA, 17-HDHA, and 19,20-DiHDPA), in combination with other oxylipins included in the panel, in their free form or nano/micro-encapsulated as a supplement to OMM, donor HM, or infant formula preparations, are beneficial in preventing intestinal disorders and achieving proper maturation of the digestive system in PIs.

The generation of oxylipin-loaded vesicles at industrial level for commercialization or for conducting clinical trials is more accessible and more reproducible than the isolation of HM-derived sEVs. Accordingly, the identification of certain oxylipins responsible for the therapeutic effect of the HM-derived sEVs represents a very relevant contribution to the state of the art by allowing the scaling up and commercialization of beneficial products in the treatment and prevention of intestinal disorders in PIs.

## Brief description of the drawings

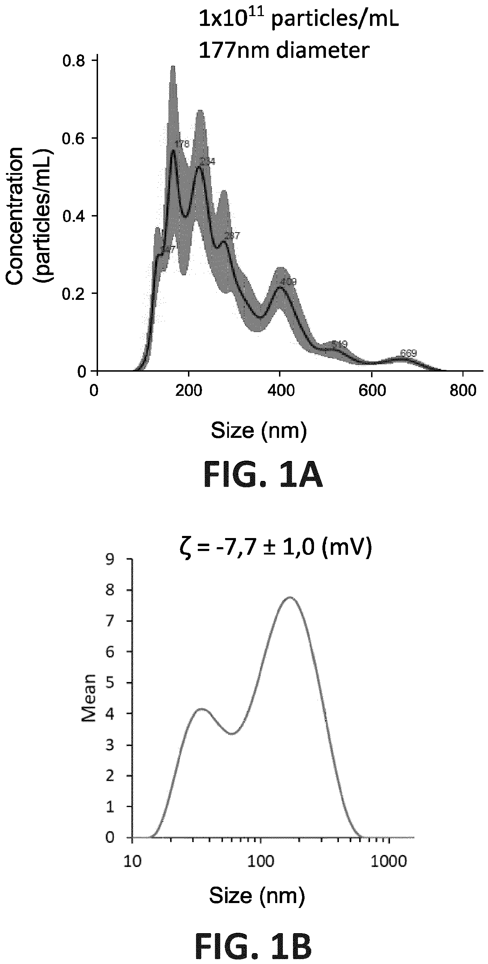

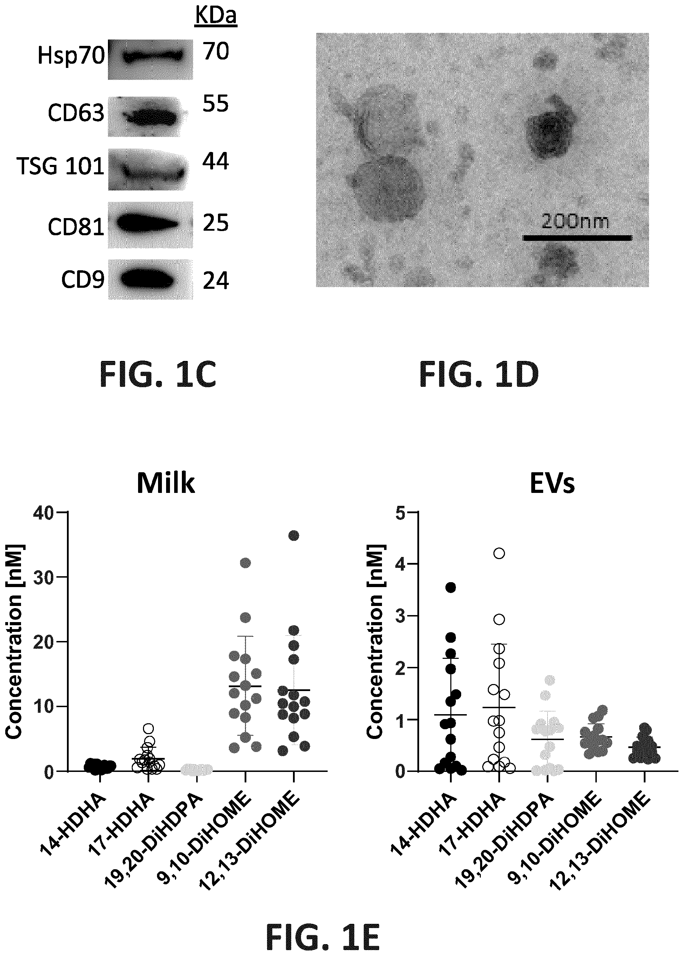

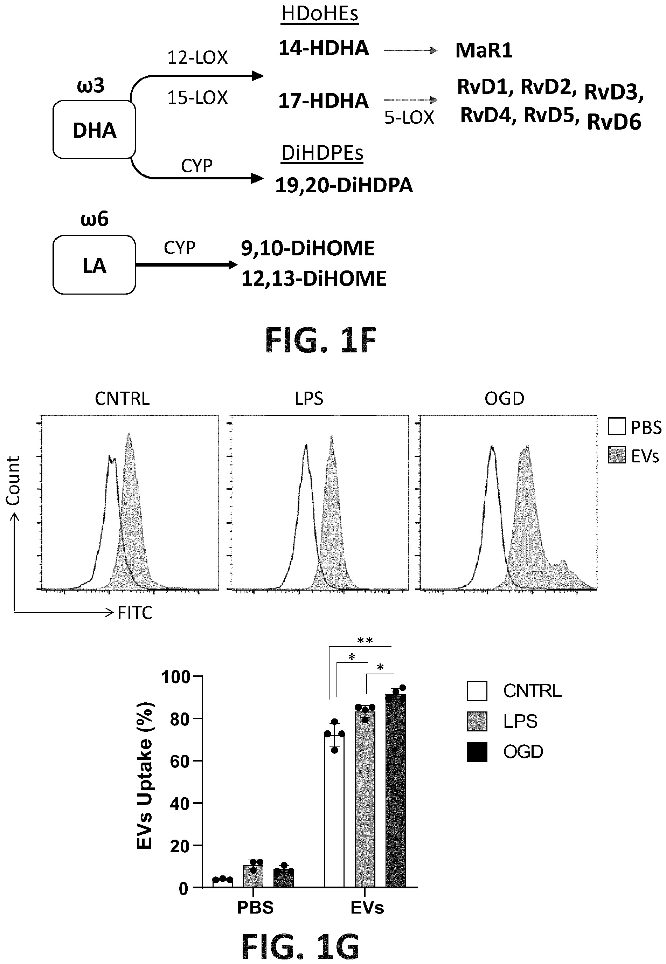

**Figure 1. Characterization of HM sEVs and oxylipins content. (A)** Representative images of sEVs assessed by nanoparticle tracking and **(B)** DLS analysis; **(C)** representative Western blots of Hsp70, CD63, TSG101, CD81 and CD9 proteins in 30 μg of loaded sEVs; **(D)** representative transmission electron microscopy images of isolated HM sEVs. Scale bar: 200 nm; **(E)** concentration [nM] of the different oxylipins in HM (left) and in HM sEVs (right) isolated from 25 mL of HM; **(F)** scheme of oxylipins synthesis; **(G)** Intestinal epithelial cells were incubated with carboxyfluorescein succinimidyl ester (CFSE)-labeled HM sEVs for 3 h at 37 °C and sEVs internalization was assessed by flow cytometry. As a negative control, PBS was mixed with CFSE and added to cells in parallel. sEVs internalization was measured by fluorescence intensity and is represented as the percentage of sEVs uptake. Graphs represent mean $\pm$ SD of three independent experiments. * $p < 0.05$, ** $p < 0.01$.

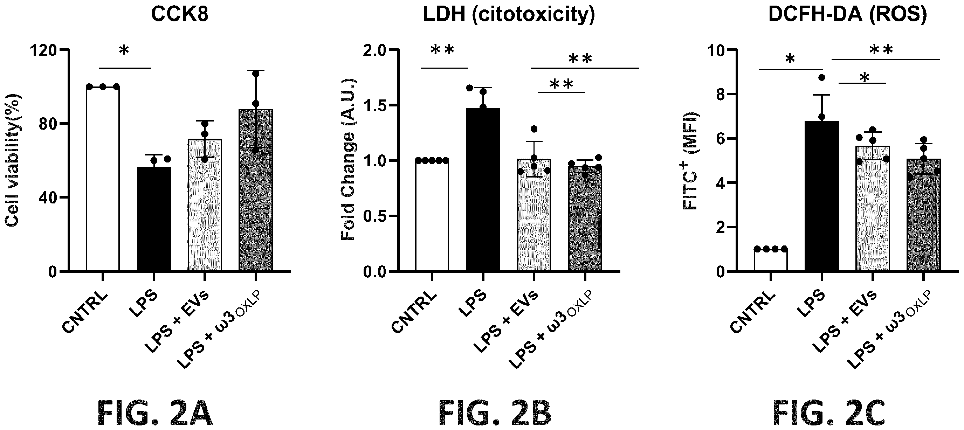

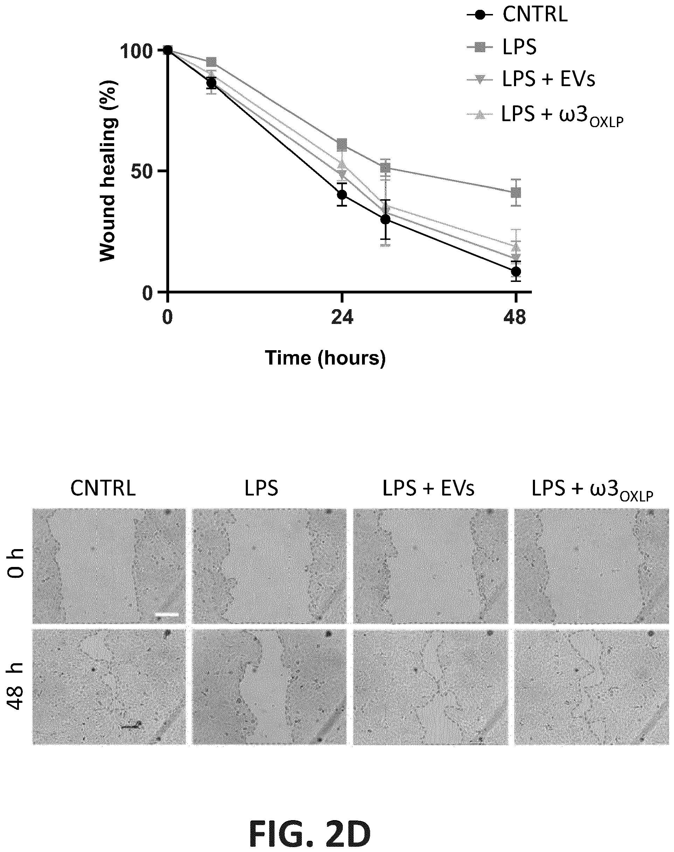

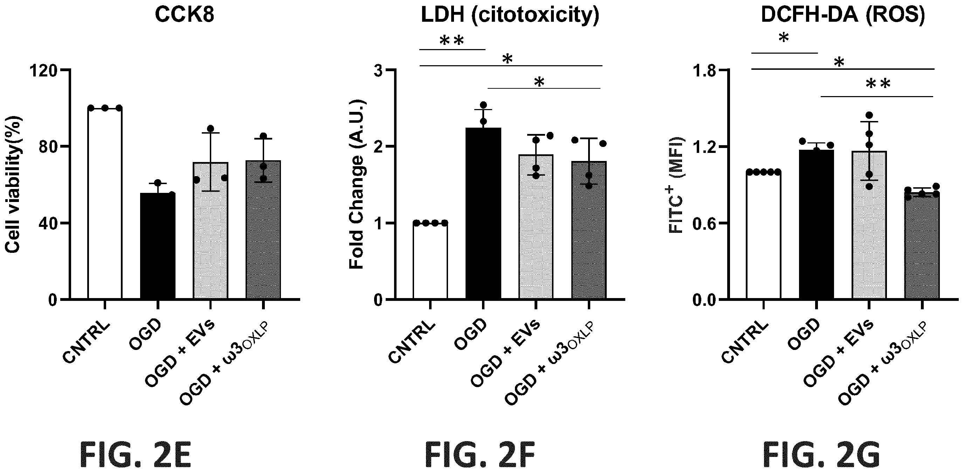

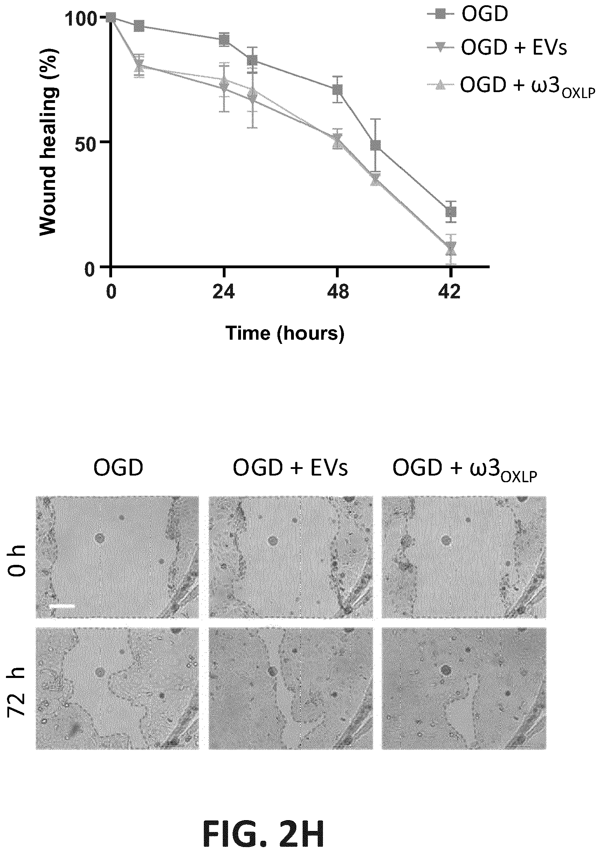

**Fig. 2. HM-derived sEVs and ω3 oxylipins protect the intestinal epithelium from damage. (A)** Quantification of cell viability of intestinal cells measured by MTT under LPS stimulation. (B) Relative quantification of lactate dehydrogenase enzyme activity (LDHA) measured by absorbance in intestinal cells under LPS stimulation. **(C)** Quantification of ROS production by oxidation of DCFH-DA measured by fluorescence in intestinal cells under LPS stimulation. **(D)** Quantification of intestinal cell wound closure under LPS stimulation. **(E)** Quantification of cell viability of intestinal cells measured by MTT under OGD condition. **(F)** Relative quantification of LDHA measured by absorbance in intestinal cells under OGD condition. **(G)** Quantification of ROS production by oxidation of DCFH-DA measured by fluorescence in intestinal cells under OGD condition. Data were normalized to initial wound area and are represented as mean percentage $\pm$ SD. Representative brightfield images of wound closure assay at different times (0 and 48 or 72 h) after wound generation on a culture of intestinal cells monolayer, stimulated with LPS (100 ng/mL) or culture under OGD conditions. Images were taken at $10\times$ magnification. Experiments were performed in triplicate. * $p < 0.05$, ** $p < 0.01$.

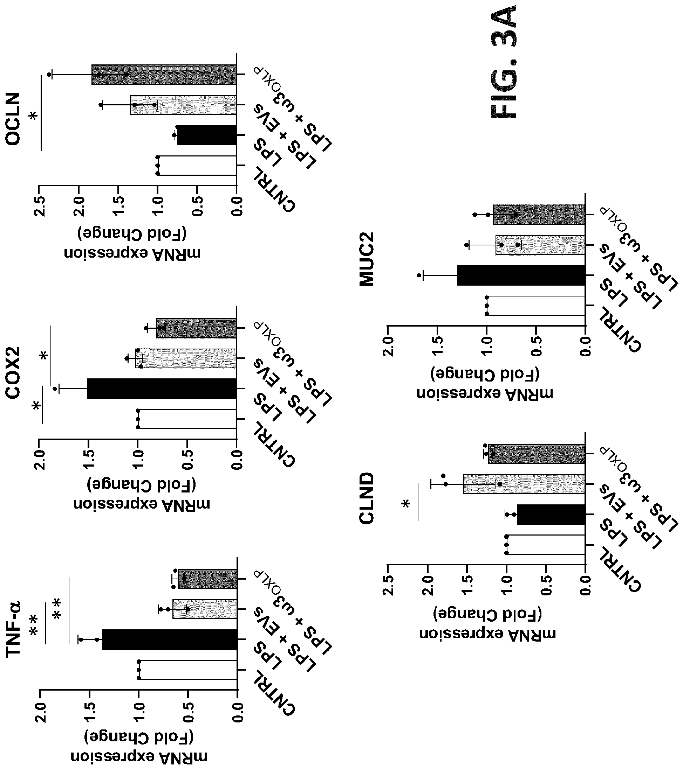

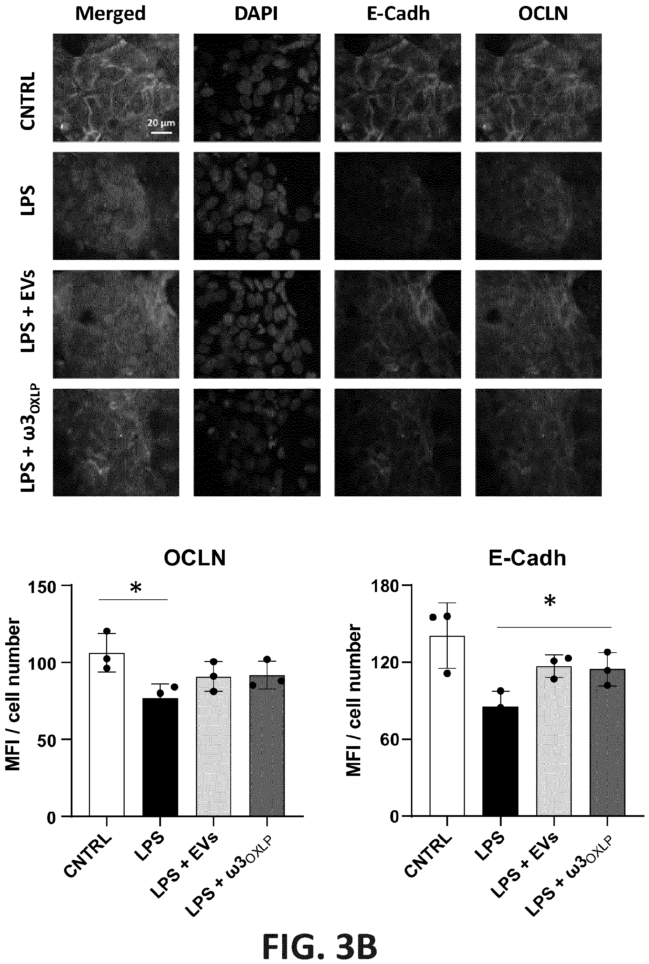

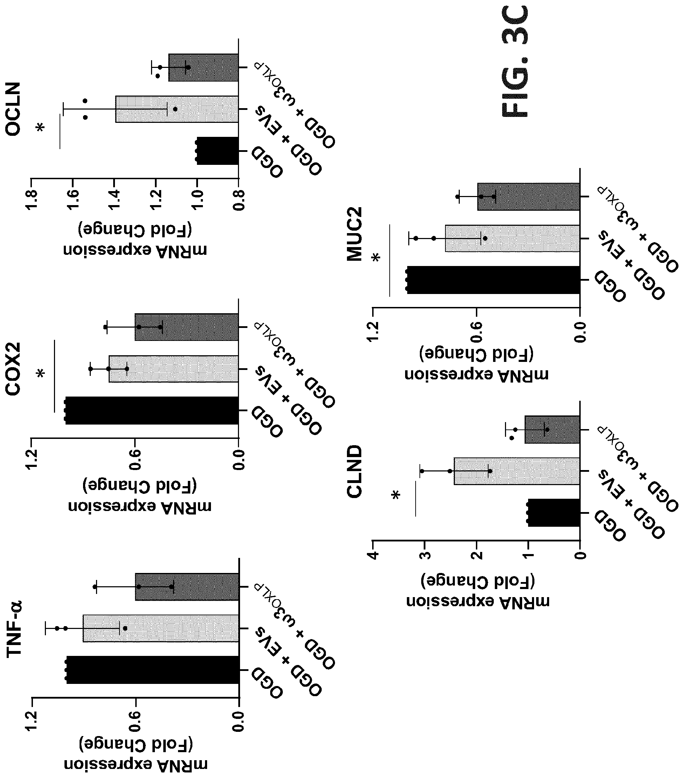

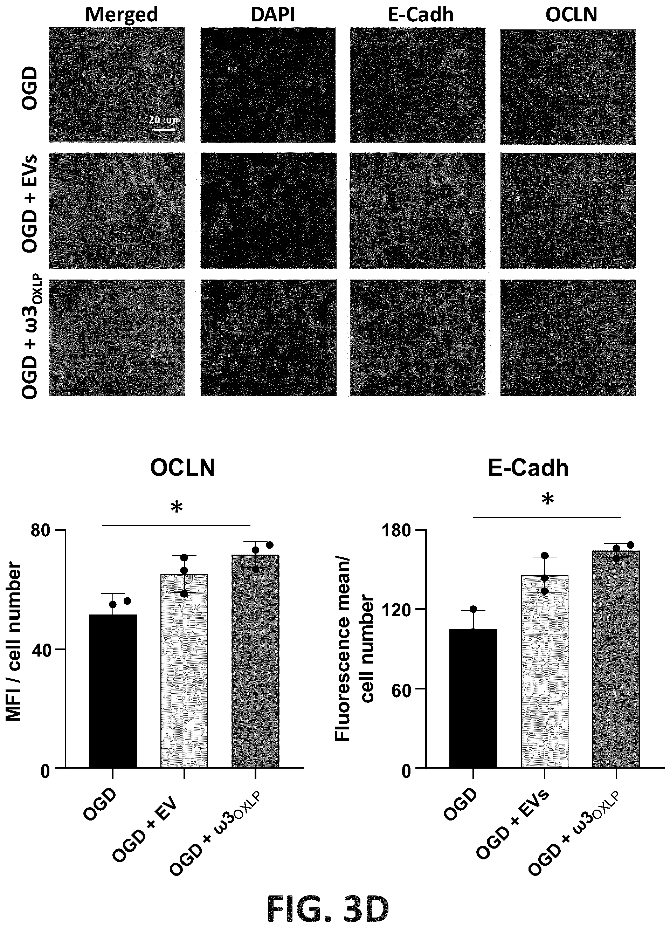

**Fig. 3. HM sEVs and ω3-oxylipins reduce inflammatory responses in the inflamed epithelium. (A)** Expression levels of TNF-$\alpha$, OCLN, CLND, COX-2 and MUC2 quantified by RT-qPCR in intestinal cells stimulated with LPS and/or treated with 7.5 $\mu$g/mL sEVs or 0.5 nM of each of the three ω3-oxylipins. **(B)** E-cadherin (E-Cadh, red) and occludin (OCLN, green) immunofluorescence and nuclei staining (blue) to show the distribution of tight junctions in the cell membrane. **(C)** Expression levels of TNF-$\alpha$, OCLN, CLND, COX-2 and MUC2 quantified by RT-qPCR in intestinal cells culture under OGD condition and/or treated with 7.5 $\mu$g/mL sEVs or 0.5 nM of each of the three ω3-oxylipins. Unstimulated intestinal cells were used as controls. The expression level of the target gene in each sample was normalized to GAPDH expression. **(D)** E-cadherin (E-Cadh, red) and occludin (OCLN, green) immunofluorescence and nuclei staining (blue) to show the distribution of tight junctions in the cell membrane. Scale bar: 20pm. The bar graph shows the quantification of the mean fluorescence intensity (MFI). The graph represents the mean $\pm$ SD of three independent experiments. * $p < 0.05$, ** $p < 0.01$

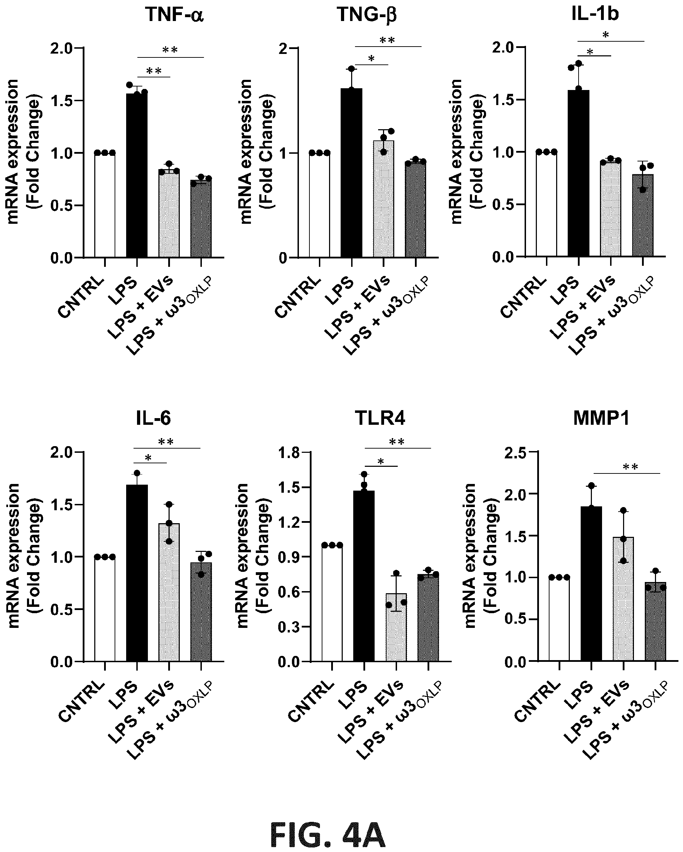

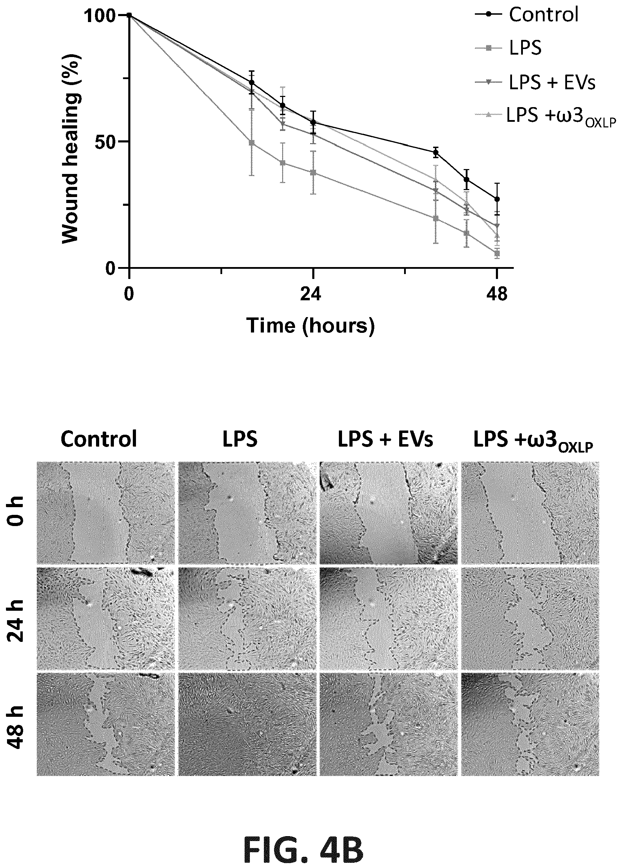

**Fig. 4. Treatment with HM sEVs and ω3-oxylipins are able to prevent LPS-induced fibrosis. (A)** Expression levels of TNF-$\alpha$, TGF-$\beta$, IL-1$\beta$, IL-6, TLR4 and MMP1 quantified by RT-qPCR in fibroblast stimulated with LPS and/or treated with 7.5 $\mu$g/mL sEVs or 0.5 nM of each of the three ω3-oxylipins. Unstimulated fibroblasts were used as controls. The expression level of the target gene in each sample was normalized to GAPDH expression. (B) Quantification of fibroblast wound closure. Data were normalised to initial wound area and are represented as mean percentage $\pm$ SD. Representative brightfield images of wound closure assay at different times (24 and 48 h) after wound generation on a monolayer fibroblast culture stimulated with LPS alone or treated with 7.5 $\mu$g/mL sEVs or 0.5 nM of each of the three ω3-oxylipins. Images were taken at 10x magnification. Experiments were performed in triplicate. * $p < 0.05$, ** $p < 0.01$.

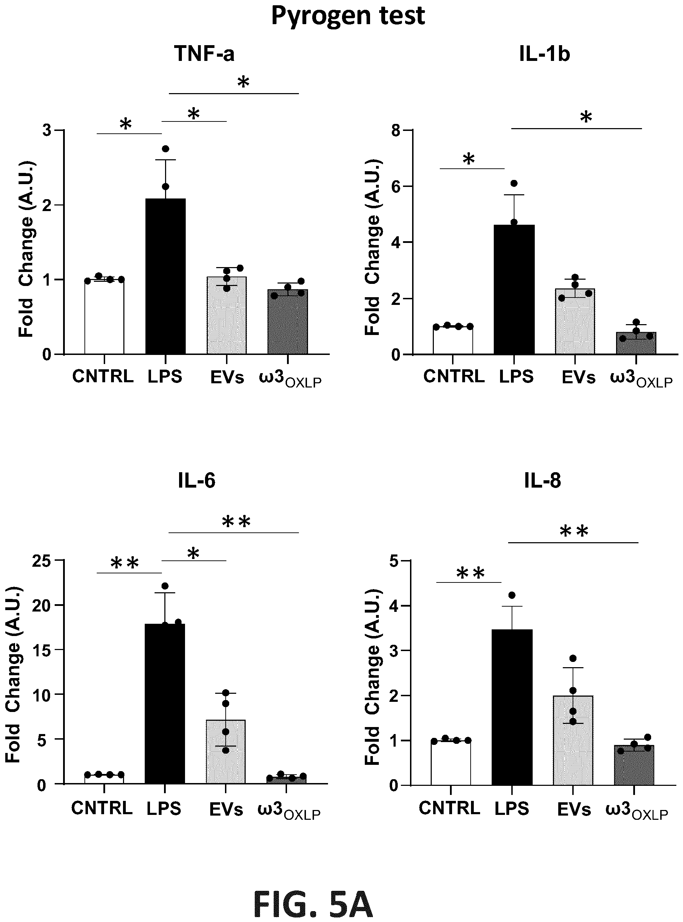

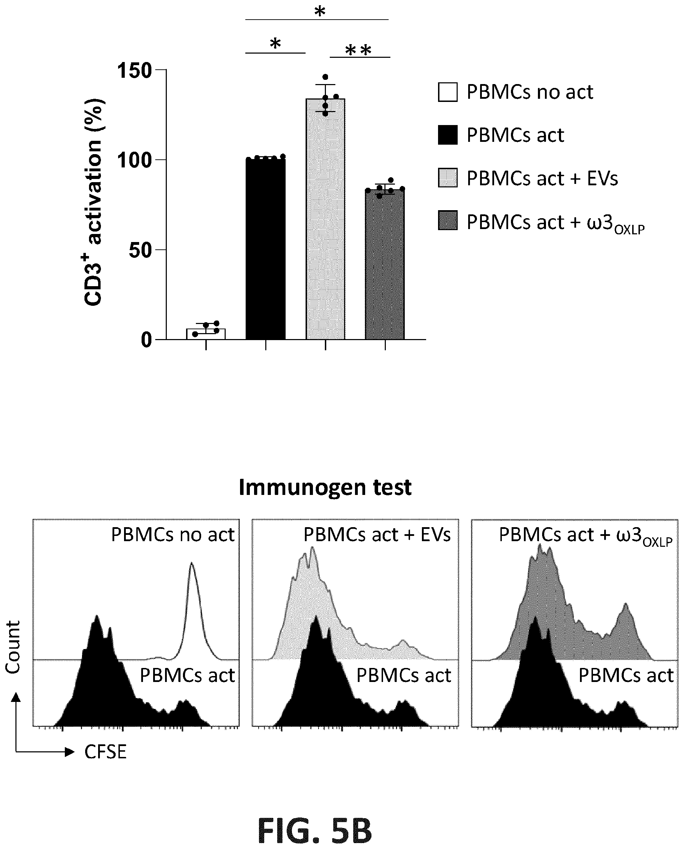

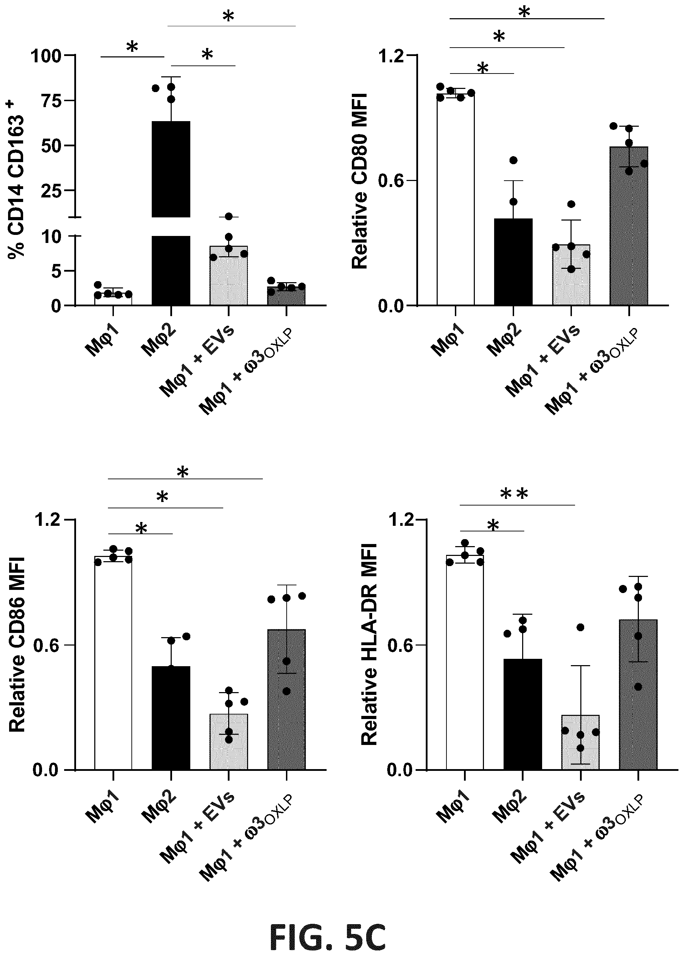

**Fig. 5. Response of HM sEVs and ω3-oxylipins on immune system cells. (A)** Expression values of proinflammatory genes (TNF-$\alpha$, IL-1$\beta$, IL-6 and IL-8) in PBMCs cultured for 6 h with or without treatment (LPS, HM sEVs and ω3-oxylipins). **(B)** PBMCs were stained with CFSE and stimulated with anti-CD3 and anti-CD28 in the presence or absence of HM sEVs or ω3-oxylipins. After 5 days, cells were stained with anti-CD3 antibody and T-cell proliferation was determined by flow cytometry measuring CFSE dilution. Suppression (percentage) was calculated from the expansion index. The graphs represent the mean $\pm$ SD of five independent experiments. **(C)** After 5 days of differentiation, the percentage of CD14+ and CD163+ cells was assessed by flow cytometry. After LPS activation, CD86, CD80 and HLA-DR expression was assessed by flow cytometry. The mean relative fluorescence intensity (MFI) was calculated by dividing all individual data by the mean expression in M $\phi$ 1. Graphs represent the mean $\pm$ SD of five independent experiments. * $p < 0.05$, ** $p < 0.01$.

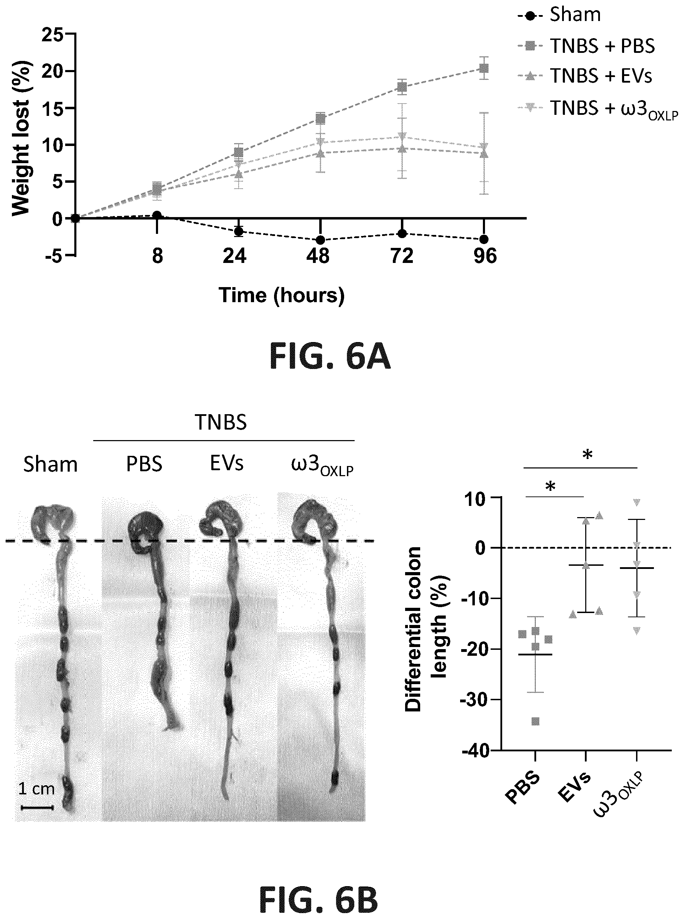

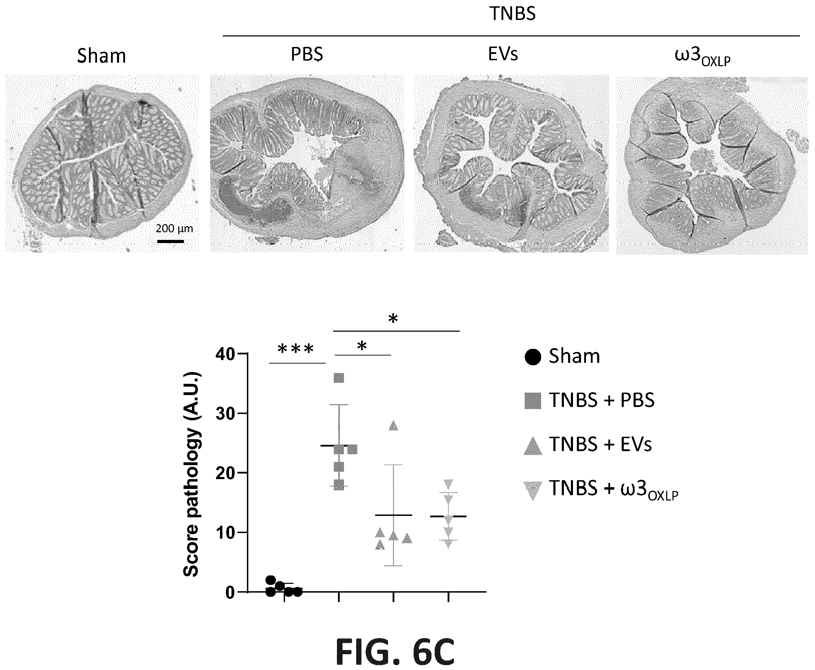

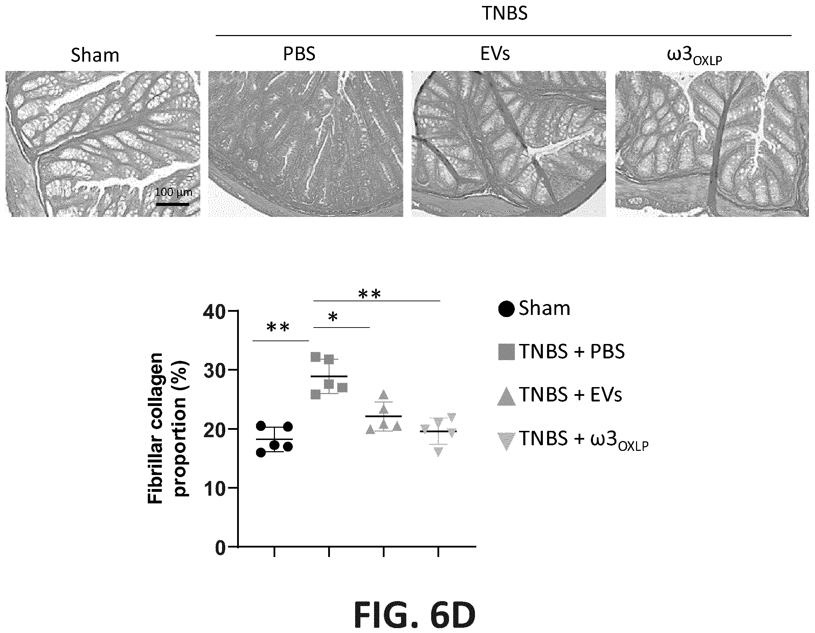

**Fig. 6. HM sEVs and ω3-oxylipins attenuate disease in mice with TNBS-induced colitis. (A)** Measured weight loss of mice throughout the experiment (4 days). **(B)** Macroscopic images of colon tissue on day 4 after TNBS administration. Scale bar: 1 cm. Percentage differential length of the colon compared to the healthy group (horizontal dotted line). Data are presented as the mean $\pm$ SD of 5 mice in each group. **(C)** Hematoxylin and eosin staining of representative histological sections of the colon of mice in the healthy group and in the PBS, sEVs and ω3-oxylipins groups after TNBS administration. Scale bar, 200 $\mu$m. **(D)** Sirius Red staining was used to detect collagen fibers. Fibrillar collagen content (%) was calculated by dividing the area stained with red by the total tissue area. The graph represents the mean $\pm$ SD of five mice. * $p < 0.05$, ** $p < 0.01$.

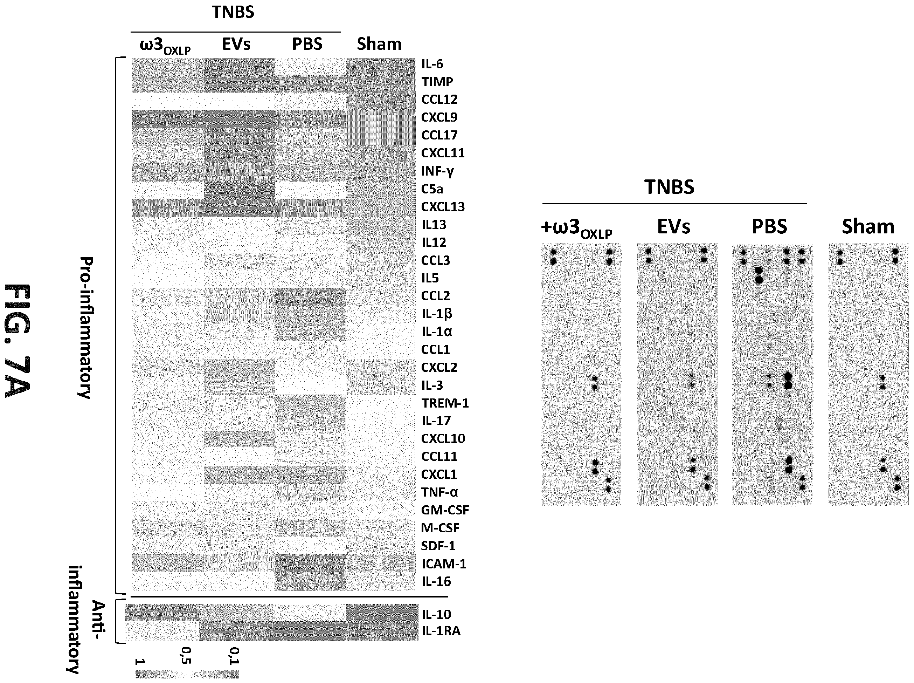

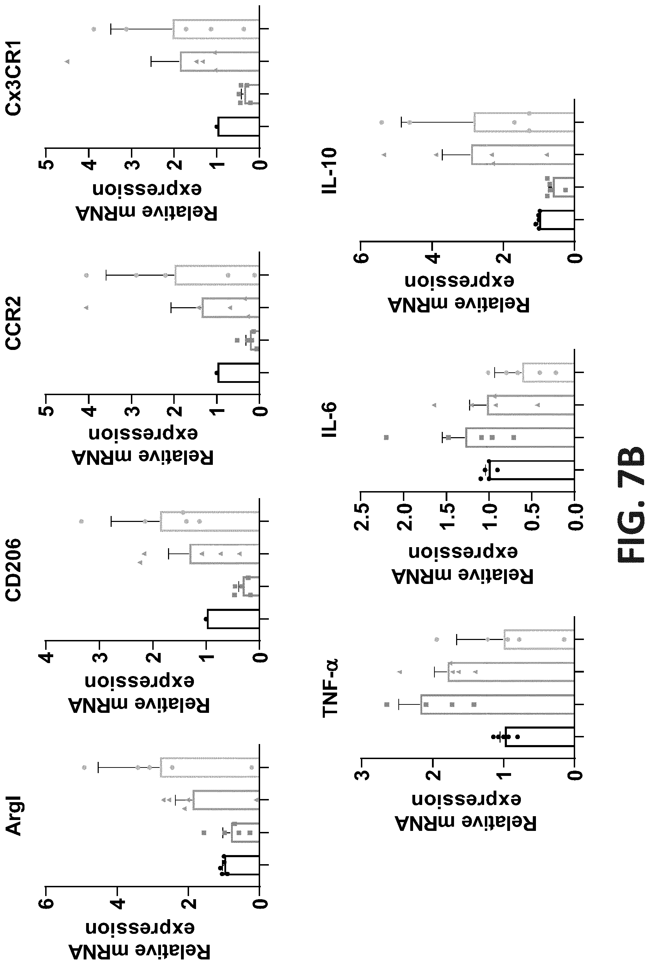

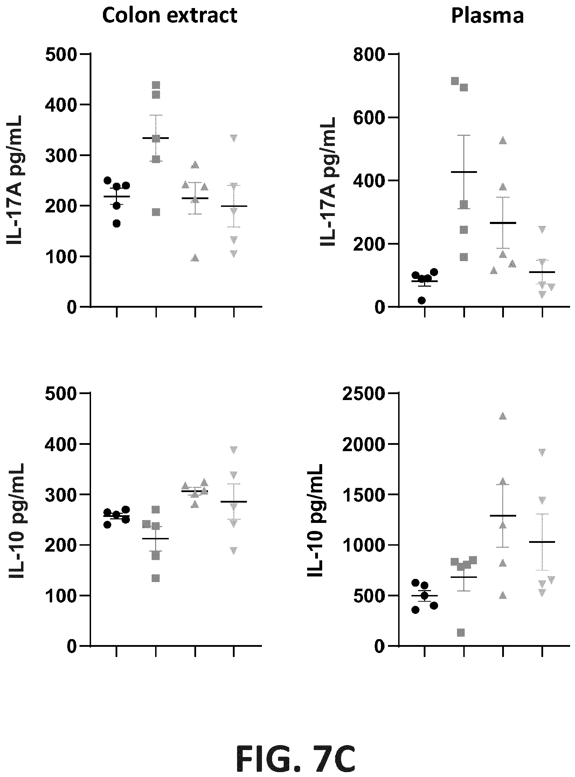

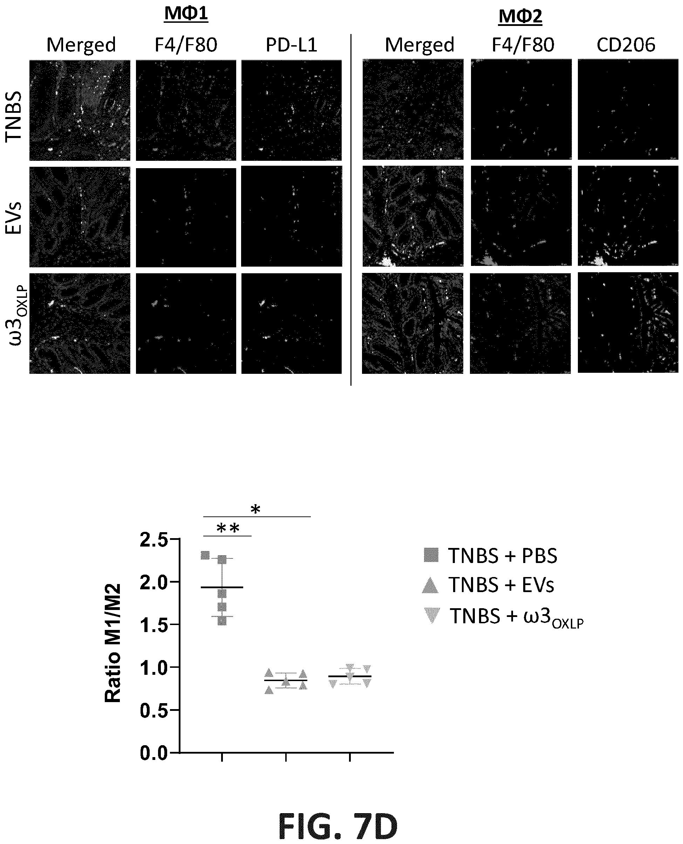

**Fig. 7. HM sEVs change the ratio of infiltrating M $\phi$ 1/M $\phi$ 2. (A)** The levels of inflammation-related cytokines were analyzed using colonic tissues by immunoblot array (left). The relative expression of each cytokine was quantified and represented with a heat map (right); data are representative of three independent experiments. **(B)** Argl, CD206, CCR2, Cx3CR1, TNF$\alpha$, IL-6, and IL-10 mRNA expression levels quantified by RT-qPCR in mouse colon. Sham group was used as control. Expression level of the target gene in each sample was normalized to $\beta$-actin expression. Graphs represent mean $\pm$ SEM of fold change of five independent experiments. **(C)** ELISA assay to assess IL-17a and IL-10 production (pg/mL) in colon extract and plasma. **(D)** Immunodetection of F4/F80 (pan-macrophage marker, red) and PD-L1 (M $\phi$ 1, green) or CD206 (M $\phi$ 2, green) in colon samples 4 days after TNBS-induced colitis. Scale bar: 100 $\mu$m. Quantification of double-positive cells per mm$^2$. Ten sections of 0.14 mm$^2$ per mouse were analyzed. Graphs represent the M $\phi$ 1/M $\phi$ 2 ratio $\pm$ SEM of five mice.

## Detailed description of the invention

Based on the needs of the state of the art, and in order to provide a commercializable product that allows taking advantage of the properties derived from the sEVs of HM in the treatment and prevention of intestinal diseases in, mainly, PIs, the authors of the present invention have identified a panel of oxylipins present in HM-derived sEVs, for which a therapeutic effect was observed.

The panel comprises the following oxylipins: 12,13-DiHOME; 9,10-DiHOME; 17-HDHA; 14-HDHA; 19,20-Di-HDPA; 14,15-DiHETRE; PGE$_2$; PGF$_{2\alpha}$; 17,18-DiHETE; 11,12-DiHETrE; 9-HOTrE; 20-HETE; 12(S)-HpETE; 12(S)-HEPE; 13-HODE; 5(S)-HEPE; 9-HODE; 15-HETE; 16(17)-EpDPE; 13-HpODE; 13-KODE; 9-HpODE; 15-HpETE; 15-KETE; 11-HETE; 9-KODE; 12-HETE; 8-HETE; 15(S)-HETrE; 9-HETE; 5-HETE; 19(20)-EpDPE; 12(13)-EpOME; 5(S)-HpETE; 9(10)-EpOME; 12-KETE; 5-KETE; 8(9)-EpETrE; and 5(6)-EpETrE.

The experiments carried out by the authors of the invention for the detection of oxylipins in samples of HM and HM derived sEVs has allowed to determine that sEVs derived from HM have a higher relative concentration of oxylipins derived from DHA (such as 19,20-DiHDPA, 14-HDHA, and 17-HDHA), for which an anti-inflammatory activity has been described, than of LA-derived oxylipins, for which pro-inflammatory activity has been described (such as 9,10-DiHOME and 12,13-DiHOME).

Therefore, in a main aspect, the present invention refers to a composition comprising the combination of the pro-resolutive and/or anti-inflammatory oxylipins 14-HDHA, 17-HDHA, and 19,20-DiHDPA (composition of the invention).

In a preferred embodiment, oxylipins 14-HDHA, 17-HDHA, and 19,20-DiHDPA are present in the composition in equal amounts.

Oxylipins 17-HDHA, 14-HDHA, and 19,20-DiHDPA (named in ahead as w3-oxylipins) are derived from the omega-3 fatty acid DHA. They are produced by the action of the lipoxygenases on DHA, and it has been shown that they have various biological activities, including anti-inflammatory and pro-resolving effects. Importantly, in addition to having their own biological effects, some of these oxylipins are precursors of SPMs: 17-HDHA is the precursor of SPM serie D-resolvins (RvD) and protectins (PD1), which has anti-inflammatory and pro-resolving effects. 14-HDHA is the precursor of SPM maresin (MaR). These SPMs are the ones that ultimately interact at the cellular level in the tissues and produce resolution of inflammation.

In other embodiments, the composition of the invention additionally comprise one or more oxylipins selected from the group consisting of: 12,13-DiHOME; 9,10-DiHOME; 14,15-DiHETrE; PGE$_2$; PGF$_{2\alpha}$; 17,18-DiHETE; 11,12-DiHETrE; 9-HOTrE; 20-HETE; 12(S)-HpETE; 12(S)-HEPE; 13-HODE; 5(S)-HEPE; 9-HODE; 15-HETE; 16(17)-EpDPE; 13-HpODE; 13-KODE; 9-HpODE; 15-HpETE; 15-KETE; 11-HETE; 9-KODE; 12-HETE; 8-HETE; 15(S)-HETrE; 9-HETE; 5-HETE; 19(20)-EpDPE; 12(13)-EpOME; 5(S)-HpETE; 9(10)-EpOME; 12-KETE; 5-KETE; 8(9)-EpETrE and 5(6)-EpETrE.

The above-mentioned oxylipins are derived from 1) AA, generated via lypooxigenase (LOX) enzymes pathway: 5-HETE, 8-HETE, 9-HETE, 11-HETE, 12-HETE and 15-HETE; cytochrome P450 (CYP) enzymes pathway: 5(S)-HpETE, 12(S)-HpETE, 15(S)-HpETE, 5-KETE, 12-KETE, 15-KETE, 5(6)-EpETrE, 8(9)-EpETrE, 11,12-DiHETrE, 14,15-DiHETrE and 20-HETE; or cyclooxygenase (COX) enzymes pathway: PGE2 and PGF2$\alpha$ (prostaglandins); from 2) LA, generated via LOX enzymes pathway: 9-HODE, 13-HODE, 9-KODE, 13-KODE, 9-HpODE and 13-HpODE; or CYP enzymes pathway: 9,10-DiHOME, 12,13-DiHOME, 9,10-EpOME and 12,13-EpOME; from 3) EPA, generated via LOX enzymes pathway: 5(S)-HEPE and 12(S)-HEPE; or CYP enzymes pathway: 17,18-DiHETE; from 4) $\alpha$-LA, generated via LOX enzymes pathway: 9-HOTrE; from 5) Dihomo-$\gamma$-Linoleic Acid (DGLA), generated via LOX enzymes pathway: 15-HETrE and, finally, from 6) DHA, generated via CYP enzymes pathway: 16(17),EpDPE and 19(20)-EpDPE.

Oxylipins included in the composition of the invention mimic the oxylipin profile present in HM sEVs. Therefore, oxylipins of the invention can be obtained from HM sEVs, another natural source or by synthesis, which facilitates their scaling and commercialization.

Fatty acids and lipids are unstable when incorporated into the food matrix, which promotes their degradation before they can be absorbed by the body. For this reason, in a preferred embodiment, oxylipins included in the composition are previously nano or micro-encapsulated, either each oxylipin individually or the combination or three or more oxylipins together.

In a particular embodiment, oxylipins can be nano/micro-encapsulated using one or a combination of two of the following methods: spray drying, complex coacervation or microfluidic techniques.

In spray drying method, the lipids are homogenised with the encapsulation material to form an emulsion, which is atomised in a drying chamber at a predefined flow rate. When water is removed from the emulsion by applying thermal energy, the coated dry active ingredient is obtained with a particle size between 10-400 μm, more stable to moisture, oxygen, light and other environmental stress factors.

In the complex coacervation method, the emulsified lipid is homogenised with two polymers of opposite charge and pH is adjusted to form the outer coating of the capsule.

The optimum encapsulation material in both methods are whey proteins as they are very stable under the physical and chemical conditions of the final food product. However, other materials could also be used: egg proteins, soy proteins, starch, vegetable chitosan, pea albumin, pea globulin, sucrose or maltodextrin.

Another chosen encapsulation method is the formation of lipid-based nanoparticles using microfluidic technique. They are mainly composed of a cationic/ionisable lipid and auxiliary lipids such as phospholipids, cholesterol and/or polyethylene glycol) (PEG) lipids.

The nano/micro-encapsulation of oxylipins allows the use of lower doses and increases the absorption of the compounds at the intestinal level.

The administration of said oxylipins can be done as part of a nutritional intervention or as a preventive or therapeutical intervention in subjects in need thereof. Therefore, in another embodiment, the composition can be manufactured as a pharmaceutical composition, a nutritional supplement, a nutraceutical product, a medical food composition, or a nutritional composition.

In a preferred embodiment, the composition of the invention is used as a nutritional supplement for adults or infants. Additional ingredients may be included in the production of complete nutritional supplements. Thus, the supplements may improve other active agents, preservatives, buffering agents, salts, carriers, excipients, diluents, or other ingredients that may be administered by oral route.

In a particular embodiment, the oxylipins can be added, both in free form or nano/micro-encapsulated, as a nutritional supplement to OMM, donor HM (DHM) or infant formula. Infant formula can either be in the form of a liquid, ready-to-consumer or concentrated, or in the form of a dry powder that may be reconstituted to form a formula upon addition of water. Such formulae are well-known in the art.

In a preferred embodiment, the composition of free or nano/micro-encapsulated oxylipins is provided as a nutritional supplement to PIs at risk of NEC, added to OMM, DHM, or infant formula.

As shown in the examples below, ω3-oxylipins have similar pro-resolving and therapeutic effects than HM sEVs, restoring intestinal barrier cohesion, protecting intestinal epithelial cells from damage by inflammation or hypoxia, preventing LPS-induced fibrosis, etc. Further, ω3-oxylipins promote an anti-inflammatory environment in intestinal alterations, preventing the inflammatory response in intestinal diseases by regulating macrophage infiltration and cytokine expression.

According to the above, in another embodiment, the present invention refers to a pharmaceutical composition comprising the composition of the invention for use in the prevention and/or treatment of intestinal diseases in adults or infants.

From a neonatal perspective, the intestinal disease can be selected from a range of pathologies that affect intestinal motility, such as dilation, NEC, and spontaneous intestinal perforation. These conditions can hinder the acquisition of a suitable rate of enteral feeding, leading to prolonged parenteral nutrition with associated risks including nutritional deficiencies, metabolic issues, and infections. Additionally, there is a risk of sepsis and peritonitis with disseminated intravascular coagulation in cases of NEC and spontaneous perforation, which can result in the need for resection surgery with medium and long-term consequences for the physical and neurocognitive development of premature infants.

From a paediatric perspective, pathologies can be selected from asthma, where supplementation with oxylipins can reduce inflammation and symptoms, cardiovascular diseases in children, e.g. reducing oxidative stress in pediatric patients with dilated cardiomyopathy, improving heart rate variability in obese children and reducing cardiotoxicity associated with chemotherapy in children with acute lymphoblastic leukemia; inflammatory bowel diseases, such as ulcerative colitis and Crohn's disease.

In a preferred embodiment, the pharmaceutical compositon is beneficial in preventing intestinal disorders in PIs, such as NEC, and achieving proper maturation of the digestive system in PIs.

In some embodiments, the pharmaceutical composition further comprise at least one pharmaceutically acceptable carrier and/or excipient.

Both, the nutritional supplement or the pharmaceutical composition of the invention can be delivered in a variety of forms, such as capsules, pills, tablets, a powder, emulsions, suspensions, solutions, oral sprays, soft and hard gelatin capsules, liposomes, chewable tablets, microspheres, delivery systems, orally disintegrating tablets, syrupes, softgels among other suitable dosages units known in the art to be taken orally by a subject.

The following examples illustrate the present invention:

EXAMPLES

*Materials and Methods*

1. Ethical Statements

All donors gave their informed consent for inclusion. The study was conducted in accordance with the Declaration of Helsinki, and the protocol was approved by the Ethics Committee of The Hospital La Fe Universitari i Politècnic, Valencia, Spain (Approval Number 2021-071-1 & 2022-748-1).

Animal procedures were approved by Hospital La Fe Ethics Committee (Protocol N° 2021/VSC/PEA/0060) according to guidelines from Directive 2010/63/EU of the European Parliament on the protection of animals used for scientific purposes.

2. Human Samples

HM samples were obtained from breastfeeding women (28-42 years of age) after informed consent.

Buffy coats of healthy donors were obtained from Centro de Transfusión de la Comunidad Valencia (Valencia, Spain) after informed consent.

3. Cell Culture

Caco-2 cells are epithelial cells isolated from colon tissue. Caco-2 cells were routinely maintained in Dulbecco's modified Eagle's medium (DMEM)-high glucose (Gibco, Thermo Fisher Scientific, Waltham, MA, USA) supplemented with 10% heat-inactivated fetal bovine serum (FBS, Corning, Glendale, AZ, USA) and 100 U/mL penicillin and 100 $\mu$g/mL streptomycin (P/S, Sigma-Aldrich, San Luis, MO, USA). Caco-2 cells were stimulated adding 60 $\mu$g/mL of Lipopolysaccharides (LPS) from Escherichia coli O111:B4 (60 $\mu$g/mL, Sigma-Aldrich, Darmstadt, Germany) to DMEM-high glucose supplemented with 0.5% of FBS and 1% P/S) during 24 h in the presence, or not, of HM-sEVs or oxylipins. Differentiation of Caco-2 cells was developed seeding 1x105 cells/cm2 onto 8 $\mu$m-pore size polycarbonate membrane of insert Transwell ® (Corning ® Inc., Corning, NY, USA) in completed medium. When Caco-2 cells reach confluence on Transwell ®, they start to differentiate spontaneously, and after 21 days they show dense microvilli on the apical side characteristic of small intestinal enterocytes. Fibroblasts were isolated in the laboratory from skin biopsies and were cultured in DMEM/F12 (Gibco, Thermo Fisher Scientific) supplemented with 10% FBS and 1% P/S. One day before stimulation, fibroblasts were seeded in DMEM/F12 serum-free medium supplemented with 1% P/S. Fibroblasts were stimulated with LPS (10 ng/mL) during 24 h in the presence, or not, of HM-sEVs or oxylipins in DMEM/F12 serum-free medium supplemented with 1% P/S.

Both fibroblast and Caco-2 cells were culture under Oxygen/Glucose Deprivation (OGD) procedure in some experiments. Oxygen-glucose deprivation was induced by culture with DMEM without glucose, glutamine, and phenol red (Thermo Fisher Scientific) in a chamber at 1.5% $O_2$.

PBMCs were isolated by density gradient centrifugation with Histopaque (Sigma-Aldrich, Darmstadt, Germany). Isolated PBMCs were cultured in Rosewell Park Memorial Institute (RPMI, Gibco, Thermo-Fisher Scientific, Waltham, MA, USA) supplemented with 10% FBS, 1 mM pyruvate, 2 mM glutamine and 1% P/S (all from Sigma-Aldrich).

4. sEV Isolation and Characterization

sEVs were isolated using a serial ultracentrifugation protocol (Ramos-Garcia, V. et al. (2023). Isolation and Lipidomic Screening of Human Milk Extracellular Vesicles. Methods in molecular biology (Clifton, N.J.), 2571, 177-188). Briefly, sEVs-containing were centrifuged at 3000× g for 10 min at 4 °C (Eppendorf 5804 benchtop centrifuge, A-4-62 rotor) to remove the remaining milk fat and milk fat globules. We repeated this step two times. After removing the upper fat layer, liquid was transferred into a 25-mL polycarbonate bottle and centrifugated at 10,000× rpm for 1 h at 4 °C (Hitachi CP100NX centrifuge, Beckman Coulter 50.2 Ti rotor). We repeated this step and supernatants were subsequently filtered manually through a 0.45 $\mu$m using a syringe. sEVs were then concentrated by three of rounds ultracentrifugation at 30,000 rpm for 2 h at 4 °C to pellet HM sEVs. Samples were filtered through a 0.22 $\mu$m filter to maintain sterility. sEVs protein concentration was determined with the Pierce BCA Protein Assay Kit (Thermo Fisher Scientific) to ensure that equal amounts of protein were used for experiments. sEVs were suspended in RIPA buffer (1% NP40, 0.5% deoxycholate, 0.1% sodium dodecyl sulfate in Tris-buffered saline (TBS), (Sigma-Aldrich)) for Western blotting and in PBS for characterization and functional analysis. NTA and electron microscopy were performed as described (Gonzalez-King. H. et al. "Hypoxia Inducible Factor-1 a Potentiates Jagged 1-Mediated Angiogenesis by Mesenchymal Stem Cell-Derived Exosomes" Stem Cells (2017) Epub 2017 Apr 24).

5. Western Blot Analysis

sEVs were lysed in 100 μL of RIPA buffer containing protease (Complete, Sigma-Aldrich) and phosphatase (PhosSTOP, Sigma-Aldrich) inhibitors. Equal amounts of protein samples were mixed with non-reducing Laemmli sample buffer (BioRad) and denatured at 96 °C for 5 min. Proteins were separated on 10% SDS-polyacrylamide gels and transferred to polyvinylidene difluoride membranes (Immobilon-P; Millipore). Membranes were blocked with TBS containing 5% (w/v) non-fat dry milk powder with 0.1% Tween-20. Human primary antibodies used for Western blotting were: anti-HSP70 (dilution 1/500; Cell Signaling Technology; D69), anti CD63 (dilution 1/500; Santa Cruz; H-193), anti-TSG101 (dilution 1/200; Santa Cruz; C-2), anti-CD81 (dilution 1/500; Santa Cruz; B-11) and anti-CD9 (dilution 1/500; Santa Cruz; C-4). Secondary antibodies used were anti-IgG rabbit (dilution 1/4000; Dako; P0448) and anti-IgG mouse (dilution 1/10,000; Sigma-Aldrich; A9044). Detection was carried out using peroxidase-conjugated secondary antibodies and the ECL Plus Reagent (GE Healthcare, Chicago, IL, USA) or SuperSignalTM West Femto (Thermo Fisher Scientific). Reactions were visualized using an Amershan Imager 600 (GE Healthcare) and quantified with imaged software (NIH, Bethesda, MD, USA).

6. Uptake of Labeled sEVs

To measure sEV uptake by Caco-2 cells in different conditions, first sEVs were labeled with CFSE (Thermo Fisher Scientific) as previously described *Czernek L., et al. "The Uptake of Extracellular Vesicles is Affected by the Differentiation Status of Myeloid Cells". Scand. J.*

Immunol. 2015;82:506-514. In order to stain sEVs with CFSE, 1 μL of CFSE (5 mM) stock was added to sEVs diluted in 1mL PBS and incubated for 15 min at 37 °C in darkness in a 1 mL tube. After this time, the sEVs were washed with PBS in Amicon Ultra- -0.5 Centrifugal Filter 100KDa (Merk, Darmstadt, Germany). sEVs were resuspended in 30 μL of filtered PBS and added to 1x105 Caco-2 cells seeded in a p48 well. CFSE mixed with PBS was used as a negative control to normalize the amount of unincorporated dye. After 24 h of incubation, CFSE-positive cells were detected by flow cytometry. Images were acquired with 10× objective on a Paula Cell Imager (Leica Microsystems, Wetzlar, Germany) combining phase contrast and green fluorescence.

7. Extraction of the HM-sEVs Lipid Fraction and LC-MS Lipidomic Method

The sample preparation and analysis method were adapted from Stassburg, K. et al (2015) Targeted Lipidomics of Oxylipins (Oxygenated Fatty Acids). 10.13140/RG.2.1.3943.9207. HM sEV and HM samples were extracted using a solid phase extraction (SPE) Oasis® MAX 96 well plate from Waters (Taunton, Massachusetts). Recovered sample extracts were evaporated using a miVac centrifugal vacuum concentrator from Genevac LTD (Ipswich, UK) and dissolved in 60 μL methanol:acetonitrile (50:50, v/v).

Sample extracts were analyzed employing an Acquity-Xevo TQ-XS system from Waters (Milford, MA, USA) operating in the negative electrospray ionization mode (ESI-). Separations were performed on a Waters Acquity UPLC BEH C18 (2.1 × 100 mm, 1.7 μm) column using a 0.1% v/v acetic acid and acetonitrile: isopropanol (90:10 v/v) binary gradient. MS detection was carried out by multiple reaction monitoring (MRM). Oxylipins quantified were: 12,13-DiHOME, 9,10-DiHOME, 14,15-DiHETRE, PGE2, PGF2α, 19,20-DiHDPA, 17-HDHA, 14-HDHA, 17,18-DiHETE, 14,15-DiHETE, RvD5, Maresin 2 and 8(S),15(S)-DiHETE.

Oxylipins semi-quantified were: Tetranor-PGFM; 1a,1b-dihomo-PGF$_{2\alpha}$; PGF$_{1\alpha}$; LTD4; LTE4; 15-deoxy-Δ12,14-PGD2; 12S-HHTrE; 5,6-DiHETrE; 11,12-DiHETrE; 9-HOTrE; 15(S)-HEPE; 20-HETE; 12(S)-HpETE; 12(S)-HEPE; 8,9-DiHETrE; 5(S)-HEPE; 13-HODE; 9-HODE; 15-HETE; 16(17)-EpDPE; 13-HpODE; 13-KODE; 9-HpODE; 15-KETE; 15-HpETE; 11-HETE; 9-KODE; 12-HETE; 8-HETE; 15(S)-HETrE; 9-HETE; 5-HETE; 12(13)-EpOME; 19(20)-EpDPE; 5(S)-HpETE; 14(15)-EpETrE; 9(10)-EpOME; 12-KETE; 5-KETE; 11(12)-EpETrE; 8(9)-EpETrE; 5(6)-EpETrE; 9,10,13-TriHOME; 11b-PGE$_2$; PGD$_2$; PGK$_2$; 15-keto PGF$_{2\alpha}$; 5(S),14(R)-Lipoxin B4; PGE$_1$; PGD$_1$; 11b-13,14-dihydro-15-keto PGF$_{2\alpha}$; 15-keto PGF$_{1\alpha}$; 13,14-dihydro PGF$_{2\alpha}$; 13,14-dihydro-15-keto PGE$_2$; 13,14-dihydro-15-keto PGF$_{2\alpha}$; 5(S),6(R)-Lipoxin A4; 5(S),6(S)-Lipoxin A4; 13,14-dihydro-15-keto PGF$_{1\alpha}$; 13,14-dihydro-15-keto PGD$_2$; 1a,1b-dihomo PGF$_{2\alpha}$; 14,15-LTE$_4$; Resolvin D$_1$; Resolvin E$_1$; 13,14-dihydro-15-keto PGD$_1$; PGA$_2$; Delta12-PGJ$_2$; PGJ$_2$; LTB$_5$; 11-trans LTD$_4$; bicyclo-PGE$_2$; 11-trans LTE$_4$; 10(S),17(S)-DiHDoHE; 5(S),15(S)-DiHETE; 6-trans-LTB$_4$; Hepoxilin A$_3$; 17(18)-EpETE; 5(S),6(S)-DiHETE; 14(15)-EpETE; 20-hydrpoxy PGE$_2$; Tetranor-PGEM; Delta17-6-keto PGF$_{1\alpha}$; 6-keto PGF$_{1\alpha}$; 20-carbonyl LTB$_4$; PGF$_{3\alpha}$; 6-keto PGE$_1$; 2,3-dinor-11b PGF$_{2\alpha}$; TXB$_3$; TXB$_1$; 20-hydroxy LTB$_4$; PGE$_3$; 8-iso PGF$_{2\alpha}$; (+/-) 5-iPF$_{2\alpha}$-VI; 11b-PGF$_{2\alpha}$; PGD$_3$; 9,12,13-TriHOME.

8. T-cell Proliferation Assays

A T-cell proliferation assay was performed as described *(A., Ferrin I. et al. Human mesenchymal stromal cells*

modulate T-cell responses through TNF-$\alpha$-mediated activation of NF-$\kappa$B. Eur. J. Immunol. 2014;44:480-488) . Prior to culturing, PBMCs were labeled with 5 $\mu$M carboxyfluoroscein succinimidyl ester (CFSE; Thermo Fisher Scientific) and activated with Dynabeads™ Human T-Activator CD3/CD28 (Thermo Fisher Scientific). To evaluate the immunosuppressive potential, we added 15 $\mu$g of different sEV extracts to 105 CFSE-labeled PBMCs in 0.5 mL of medium. After 5 days of culture, proliferation of T-cells was determined by flow cytometry to measure CFSE dilution. Analyses of flow cytometry data and the expansion index (EI) (Lyons A. et al. "Analysing cell division in vivo and in vitro using flow cytometric measurement of CFSE dye dilution". J. Immunol. Methods. 2000;243:147-154. doi: 10.1016/S0022-1759(00)00231-3) were performed using FlowJo software (FlowJo LLC, BD, Franklin Lakes, New Jersey, United States). Percentage of immunosuppression was calculated normalizing data to a 0-100% scale by establishing 0% immunosuppression for the EI of activated PBMCs not treated (Act) and 100% immunosuppression for the EI of non-activated PBMCs' (no Act) using the following formula:

$$\% \, Immunosuppression = \frac{(EI_{Act} - EI_{treated})}{(EI_{Act} - EI_{none \, Act})} \, x \, 100$$

### 9. Flow Cytometry

For flow cytometry analysis PBMCs were first incubated with a blocking solution (PBS containing 1% normal mouse serum) for 10 min and then incubated with saturating amounts of fluorochrome-conjugated antibodies for 1 h at 4 °C. Human antibodies used were: anti-CD3 (PerCP-Cy, BD Biosciences; SK7), anti-CD4 (BV510, BD Biosciences; L200) and anti-CD8 (PE-Cy7, BD Biosciences; RPA-T8) at concentrations recommended by manufacturers, and were analyzed using a BD FACSCANTO II flow cytometer equipped with Flowjo® software (FlowJo LLC, BD, Franklin Lakes, NJ, USA).

### 10. Cell viability assay

To test whether HM sEVs or oxylipins altered cell viability, Caco-2 cells were cultured at a density of $1 \times 104$ cells/cm$^2$ on a 96-well plate. Cells were stimulated with LPS or cultured under OGD conditions and treated with sEVs or oxylipins. After 24 h Cell Counting Kit-8 (CCK-8) assay was used to measure proliferation following the manufacturer's instructions. The optical density of the cultures was measured at 450 nm in each well 4 h after incubation with the CCK-8 assay solution.

### 11. Lactate Dehydrogenase Assay

Caco-2 cells were seeded at $1 \times 104$ cells/cm$^2$ in completed medium. Next day, cells were culture under OGD conditions during 24h, the supernatant was tested for lactate dehydrogenase using the Cytotoxicity Detection KitPLUS (LDH) (Roche, Indianapolis, IN, United States).

### 12. Oxidative Stress Assay

LPS and OGD-treated cells were washed with PBS and stained either with 5 $\mu$M DCFH-DA (2',7'-dichlorofluorescin diacetate; Sigma-Aldrich) for 20 min at 37 °C, for detect cell reactive oxygen species (ROS) and quantify the overall oxidative stress in cells. After staining, cells were washed three times with PBS to eliminate the unconjugated stain, detached with trypsin/EDTA and analyzed by flow cytometry.

### 13. Scratch Assay

Both Caco-2 cells and fibroblast were seeded in a 24-well plate at $2 \times 105$ cells/well. Caco-2 cells were stimulated with LPS and treated with sEVs and oxylipins during 48 h. To develop scratch under OGD conditions, medium was replaced after 24 h to completed medium and cells were cultured under standard oxygen culture conditions. Caco-2 cells were stimulated with LPS and treated with sEVs and oxylipins during 48 h. A straight line in the monolayer of cells was created using a 20-$\mu$L pipette tip. Images were taken until 48 h after the addition of treatments using a Leica DM600 inverted microscope at 10 $\times$ magnification. The area of the scratch wound was then measured using imaged.

### 14. Real Time Quantitative PCR

RNA was extracted using RLT buffer (Qiagen, Dusseldorf, Germany) and purified with the RNeasy Plus Mini Kit (Qiagen). RNA was quantified spectrophotometrically using a NanoDrop ND-1000 (NanoDrop Technologies, Wilmington, DE, USA). cDNA was obtained by reverse transcription using the PrimeScript RT Reagent Kit (Takara, Kusatsu, Japan). RT-qPCR was performed with the respective human-specific sense and antisense primers and RT-SYBR™ Green PCR Master Mix (Applied Biosystems). Multiwell plates of 384 wells were run on a Viia 7 PCR System (Applied Biosystems). The mRNAs evaluated were: human GAPDH (hGAPDH), hTNF$\alpha$, hIL-1$\beta$, hTLR4, hl-L6, hIL-8, hCOX2, hOCLN, hCLDN, hMUC2, hTGF$\beta$, hMMP1, mouse ArgI (mArgI), mCD206, mCCR2, mCx3CR1, mTNF$\alpha$, mIL-6 and mIL-10.

15. Immunofluorescence of Cell Cultures

Caco-2 cells were cultured on Transwell ® for differentiation. After 21 days cells were cultured under LPS or OGD conditions and treated with HM sEVs or oxylipins during 24h. Next day, cells were fixed in 4% paraformaldehyde for 10 min, washed three times with PBS and permeabilized and blocked with BSA 5% and 0.1% Triton X-100 in PBS for 1 h. Cells were then incubated with mouse anti-human Occludin (Santa Cruz, E-5) and rat anti-human E-cadherin (EMD Millipore, DECMA-1) at a concentration of 1/200 in a humidified incubator overnight. We used goat anti-mouse IgG (1:500, Alexa Fluor® 488, Abcam) and goat anti-rat IgG (1:500, Alexa Fluor® 555, Abcam) as secondary antibodies. Nuclei were stained with DAPI. Quantification of MFI was performed using imaged.

16. Pyrogen test assay

In vitro pyrogen test using PBMCs was applied to detect substances that activate human immune cells to release pro-inflammatory cytokines such as TNF$\alpha$, IL-1$\beta$, IL-6 and IL-8 by qPCR. PBMCs ($4 \times 106$ cells/mL) were incubate with HM sEVs and oxylipins for 5h. LPS at concentration of 1$\mu$g/mL was used as positive control.

17. Mice

Adult male Balb/c mice (6 weeks old, 22-26 g) were purchased from Envigo (Inotiv, Inc., Indianapolis, Indiana, USA), and maintained under standard laboratory conditions. All animal procedures were approved by institutional ethical and animal care committees.

18. TNBS-induced Colitis

TNBS colitis was induced by an intrarectal administration of 100 $\mu$L of TNBS (3.5 mg per 20 g mice, Sigma-Aldrich) dissolved in 40% ethanol, as previously described [Cosín-Roger J. et al. "The activation of Wnt signaling by a STAT6-dependent macrophage phenotype promotes mucosal repair in murine IBD". Mucosal Immunol. 2015;9:986-998). Vehicle-treated mice received 100 $\mu$L of 0.9% NaCl dissolved in 40% ethanol. Some mice were treated 6 h after TNBS induction oral with HM sEVs in 100 $\mu$L of PBS, oxylipins preparation or only PBS. Mice were killed by cervical dislocation on day 4 after TNBS administration. Colon length was measured and colon tissue was frozen in liquid nitrogen for RNA extraction and fixed in 4% paraformaldehyde acid and embedded in paraffin for immunohistochemistry.

19. Production of Oxylipins Preparation for In Vivo Assays

For the in vivo assay of oxylipins, a preparation of them, using bovine serum albumin (BSA) as a carrier, was done. It was prepared in PBS with 10% fatty acid-free BSA (FAF-BSA, Sigma-Aldrich) to a 10 mM stock concentration. FAF-BSA was mixed with PBS and stirred for 3 h at room temperature and then filtered through a 0.22-$\mu$m filter. Finally, 14 HDHA, 17 HDHA, and 19-20 DiHDPA stocks were added and stirred again for 16 h at 37 °C. Oxylipins preparation was freshly prepared before experiment.

20. Mouse Histology and Immunofluorescence

Paraffin-embedded colon samples were cut into 5 $\mu$m-thick sections and stained with hematoxylin-eosin (Sigma-Aldrich) to evaluate inflammatory infiltrates, the presence of ulceration and the lesion of crypts. In addition, a blind pathological examination was carried out on the slides and tissues were scored using the histological colitis scoring method. This score tests for three tissue characteristics: inflammation severity (grade 0 = none, grade 1 = mild, grade 2 = moderate and grade 3 = severe), crypt damage (grade 0 = none, grade 1 = basal 1/3 damaged, grade 2 = basal 2/3 damaged, grade 3 = crypts lost and surface epithelium present, and grade 4 = crypts and epithelium lost and colon wall thickness (grade 0 = normal, grade 1 = medium, grade 2 = large and grade 3 = very large), all three relativized to the percent involvement (grade 0 = 0%, grade 1 = 1-25%, grade 2 = 26-50%, grade 3 = 51-75%, and grade 4 = 76-100%).

Score pathology is calculated as the sum of each characteristic multiplied by the percent involvement. The total maximum score is 40 (Table 1).

Table 1. Score of pathology

| SCORE OF PATHOLOGY | | | | | |
|---|---|---|---|---|---|
| **Severity of Inflammation** | | **Crypt Damage** | | **Colon wall thickness** | |
| None | 0 | None | 0 | Normal | 0 |
| | | Basal 1/3 damaged | 1 | | |
| Slight | 1 | Basal 2/3 damaged | 2 | Medium | 1 |
| Moderate | 2 | Crypts missing and surface epithelium present | 3 | Large | 2 |
| Severe | 3 | Crypts and epithelium missing | 4 | Very large | 3 |
| PERCENTAGE OF DAMAGE | | | | | |
| 1 – 25 % | | 1 | 51 – 75 % | | 3 |
| 26 – 50 % | | 2 | 76 – 100 % | | 4 |

A Picro-Sirius Red stain (Direct Red 80 and Picric Acid, Sigma-Aldrich) was used to visualize fibrosis tissue. Slides were visualized on a Leica DMD108 Digital Microscope (Leica Microsystems). For immunofluorescence, slides were blocked with 5% normal goat serum and 0.1% Triton X-100 in PBS for 1 h. Slides were incubated with rat anti-F4/F80 (dilution 1/200, Abcam, ab6640), rabbit anti-CD206 (dilution 1/200; Abcam, ab64693) or rabbit anti-CD274 (dilution 1/200, AB Clonal A11273) overnight in a humidified chamber at 4 °C and then slides were washed three times for 5 min each in PBS. Slides were incubated with anti-rat IgG Alexa 555 or anti-rabbit IgG Alexa 488 secondary antibodies for 1 h and then washed three times for 5 min in PBS. Cell nuclei were stained with DAPI and slides were mounted using FluorSave™ Reagent (Merck Millipore). The sections were observed and visualized under fluorescent microscope Leica DM2500 (Leica Microsystems). Final image processing and quantification were performed using imaged software by counting green and red spots in the fixed area.

21. Cytokine array

Colons samples were homogenized in PBS containing 1% Triton X-100 and protease inhibitor cocktail. Once centrifuged, protein amount was quantified using the Bradford assay. Protein content was normalized and analyzed with the Mouse Cytokine Array Panel A (ARY006; R&D system) according to the manufacturer's instructions. Membranes were visualized using an Amershan Imager 600 (GE Healthcare) and a semi-quantitative analysis of the comparative intensity of the spots was quantified with imaged software (NIH, Bethesda, MD, USA).

22. Statistical Analysis

Data are expressed as mean $\pm$ SD or SEM, as specified. Student's t test was used for unpaired samples in between-group comparisons. One-way analysis of variance (ANOVA) was used to compare means of more than 2 groups. Two-way ANOVA was used to simultaneously evaluate the effect of two factors on a response variable. Analyses were conducted with GraphPad Prism 8 software (San Diego, CA, USA). Differences were considered statistically significant at $p < 0.05$ with a 95% confidence interval.

*Results*

Isolation and characterization of HM sEVs

sEVs were isolated from HM by sequential centrifugation and filtration. The mean diameter observed of the purified sEVs was 150-200 nm, as determined by nanoparticle tracking analysis (NTA) (Figure 1A). Another technique, the dynamic light scattering (DLS) method, was applied to measure the size of vesicles, the $\zeta$ potential that gives an indication of the potential stability of the colloidal system and the polydispersity index (PDI) used to characterize the size distribution of sEVs. The $\zeta$ potential was -7.7 $\pm$ 1.0 mV. Note that if all the particles in suspension have a large negative $\zeta$ potential then they will tend to repel each other and there will be no tendency for the particles be added. The PDI was 0.291, revealing a relatively even size distribution of sEVs (Figure 1B). Western blot analysis revealed that the sEVs expressed the typical exosome markers Hsp70, CD63, TSG101, CD81 and CD9 (Figure 1C). Transmission electron microscopy (TEM) analysis of sEVs revealed a round or cup-shaped morphology and the size was consistent with the observed NTA-measured diameter (Figure 1D). These results demonstrated an effective isolation and accurate characterization of HM sEVs using different methods.

HM derived sEVs have a high concentration of pro-resolving oxylipins.

The quantification of oxylipins was validated by LC-MS method for HM samples and HM sEVs. Of the different oxylipins that were included in the method, the following could be quantified 12,13-DiHOME, 9,10-DiHOME, 14,15-DiHETRE, PGE$_2$, PGF$_{2\alpha}$, 19,20-DiHDPA, 17-HDHA, 14-HDHA, and 17,18-DiHETE both in HM and HM sEVs samples and 14,15-DiHETE only in HM sample (Table 2), being the most abundant 9,10-DiHOME, 12,13-DiHOME, 19,20-DiHDPA, 14-HDHA and 17-HDHA in both sample types (Figure 1E). Contrary to what is observed in HM samples, HM-derived sEVs have a higher concentration of DHA-derived oxylipins, for which anti-inflammatory activity has been described (19,20-DiHDPA, 14-HDHA, and 17-HDHA), than LA-derived oxylipins, for which pro-inflammatory activity has been described (9,10-DiHOME and 12,13-DiHOME) (Figure 1E and F). Based on these results, it was tested whether part of the protective effect of HM-derived sEVs can be attributed to the presence of these three $\omega$3-derived oxylipins: 19,20-DiHDPA, 14-HDHA, and 17-HDHA.

**Table 2.** Calibration range, linear coefficient of determination (R$^2$), limit of detection (LOD), limit of quantification (LOQ), mean concentration in HM, and mean concentration in HM sEVs of quantified oxylipins.

| Oxylipin | Calibrated range (nM) | R$^2$ | LOD (nM) | LOQ in sample (nM) | HM Mean ± SD (nM) | HM sEVs Mean ± SD (nM) |
|---|---|---|---|---|---|---|
| 9,10-DiHOME | 0.29 - 300 | 0.998 | 0.10 | 0.12 | 20 ± 30 | 0.7 ± 0.3 |
| 12,13-DiHOME | 0.15 - 300 | 0.997 | 0.05 | 0.06 | 13 ± 8 | 0.5 ± 0.2 |
| 17-HDHA | 0.3 - 300 | 0.996 | 0.02 | 0.03 | 2 ± 2 | 1.2 ± 1.2 |
| 14-HDHA | 0.15 - 300 | 0.996 | 0.4 | 0.5 | 0.7 ± 0.3 | 1.1 ± 1.1 |
| 19,20-DiHDPA | 0.07 - 300 | 0.995 | 0.02 | 0.03 | 0.23 ± 0.12 | 0.6 ± 0.5 |
| 14,15-DiHETrE | 0.07- 300 | 0.994 | 0.02 | 0.03 | 0.2 ± 0.2 | 0.04 ± 0.02 |
| 17,18-DiHETE | 0.15 - 300 | 0.994 | 0.10 | 0.12 | 0.04 ± 0.03 | 0.10 ± 0.07 |
| PGF$_{2\alpha}$ | 0.07 - 300 | 0.995 | 0.20 | 0.2 | 0.08 ± 0.05 | 0.04 ± 0.02 |
| PGE$_2$ | 0.07 - 300 | 0.995 | 0.05 | 0.06 | 0.02 ± 0.03 | 0.005 ± 0.005 |
| 14,15-DiHETE | 0.07 - 300 | 0.997 | 0.02 | 0.03 | 0.02 ± 0.02 | < LOQ |

In addition, the following oxylipins were detected in HM and HM-derived sEVs: 11,12-DiHETrE; 9-HOTrE; 20-HETE; 12(S)-HEPE; 13-HODE; 5(S)-HEPE; 9-HODE; 15-HETE; 16(17)-EpDPE; 13-HpODE; 13-KODE; 9-HpODE; 15-HpETE; 15-KETE; 11-HETE; 9-KODE; 12-HETE; 8-HETE; 15(S)-HETrE; 9-HETE; 5-HETE; 19(20)-EpDPE; 12(13)-EpOME; 5(S)-HpETE; 9(10)-EpOME; 12-KETE; 5-KETE; and 8(9)-EpETrE. Furthermore, some oxilipins were detected exclusively in HM (i.e., 15(S)-HEPE; 11(12)-EpETrE; and 14,15-DiHETE), while some were detected exclusively in HM-derived sEVs (i.e., 12(S)-HpETE and 5(6)-EpETrE).

$\omega$3-oxylipins and HM sEVs have a similar effect in protecting intestinal epithelial cells from damage by inflammation or hypoxia

It is known that the main risks for developing NEC are due to a weak immune system what increases the presence of infection or lack of blood flow that reach the colon and supplies intestinal cells with oxygen and nutrients preventing their maturation. Intestinal epithelial cells (Caco-2) were stimulated with Lipopolysaccharide (LPS) at 70 $\mu$g/mL to simulate a bacterial infection or cultured with oxygen-glucose deprivation (OGD) medium to reproduce a hypoxia situation. To know if sEVs where taken up from intestinal cells and how they affect the damage conditions, an internalization assay with CFSE-stained HM sEVs was developed. It was observed that 3 hours after sEVs addition, 72.3 ± 5.5% of intestinal cells were able to take up de HM sEVs, this uptaken was increased under LPS and OGD conditions in a 11.2% and 19.3% respectively (Figure 1G). Both LPS and OGD stimulus leads to increased cell death because of an increase of cytotoxicity and oxidative stress (ROS) in cells. To prove the protective effect of sEVs and w3-oxylipins, intestinal epithelial cells were treated with 7.5 μg/mL of HM sEVs or 0.5 nM of each of the three oxylipins. It was demonstrated that HM-derived sEVs and also w3-oxylipins protect the intestinal epithelium from damage caused by bacterial infection, improving cell viability in a 15,2% and 31,4% respectively (Figure 2A). Treatment with HM sEVs and w3-oxylipins also reduced cytotoxicity significatively (31.2% and 35.6%, Figure 2B) and oxidative stress (16.5% and 25,3%, Figure 2C). Furthermore, the effect of HM sEVs and w3-oxylipins under OGD conditions were analysed and both treatments reduce cell death (16% and 17%, Figure 2E) and cytotoxicity (15,8% and 19,5%, Figure 2F), respectively. With respect to oxidative stress assay, only ω3 oxylipins treatment reduced significatively the damage generated by hypoxia in 28,4% (Figure 2G). As a consequence of the damage mentioned above caused by LPS and hypoxia to the intestinal epithelium cells, migration of these cells is also affected. One of the main problems of Pls with NEC is the presence of "wounds" in the intestine due to lack of tissue maturation. These wounds must be rapidly repaired or else result in a worse prognosis for the baby. To investigate whether HM sEVs and ω3-oxylipins could modulate the migration of intestinal epithelial cells, an *in vitro* scratch-wound assay was developed. It was observed that LPS and hypoxia causes a decrease in wound closure. However, treatments with HM sEVs or w3-oxylipins, by reducing the cell damage caused by LPS or hypoxia, are able to restore the intestinal cells' migratory capacity and proliferation rate, promoting the development of a continuous cell layer (Figure 2D and H).

### ω3-oxylipins and HM sEVs decrease inflammatory signaling pathway produced by LPS or hypoxia and restore intestinal barrier cohesion

The inflammatory response triggered by LPS or hypoxia in the intestinal epithelium is observed in the upregulation of pro-inflammatory genes such as TNF-α and COX-2. The inflammatory stimulus triggered by LPS or hypoxia also promotes overexpression of the MUC2 (mucin 2) gene by intestinal cells in order to prevent pathogen access to the underlying mucosa. Treatment with 7.5 μg/mL of HM sEVs or 0.5 nM of each of the three w3-oxylipins has shown to reduce the expression of these genes significatively compared to LPS (Figure 3A and C). As discussed above, disruption of the intestinal barrier is one of the main risk factors for neonates suffering from NEC. In the intestinal epithelium, there are tight junctions that connect neighboring cells to each other in a way that creates a barrier preventing the free flow of substances between cells. These tight junctions are made up of proteins such as occludins (OCLN) and claudins (CLND). It was observed that stimulation of the intestinal epithelium with LPS or hypoxia decreases the expression of these genes (occludin and claudin). However, treatment with HM sEVs or w3-oxylipins is able to increase their expression (Figure 3A and C). This result was also validated by immunofluorescence. LPS and hypoxia reduced the expression of tight junction proteins (E-caderin in red and occludin in green), while treatment with HM sEVs or w3-oxylipins increased their expression and partially restored the architecture and cohesion of the intestinal epithelium (Figure 3B and D).

### ω3-oxylipins and HM sEVs are able to prevent LPS-induced fibrosis

Fibrosis is a pathological feature of most chronic inflammatory diseases, where fibroblast proliferation and migration lead to excess fibrous connective tissue, reducing its functionality. LPS stimulation activate the fibrotic condition on fibroblast, modulating the release of inflammatory cytokines and increasing their proliferation and migration. Levels of pro-inflammatory cytokines, such as TNF-α, TGF-β, IL-1 and IL-6 increased significantly 24 h after LPS stimulation. It was observed that treatment with 7.5 μg/mL of HM sEVs or 0.5 nM of each of the three w3-oxylipins significantly decreased the expression of these genes. In addition, the levels of other classic pro-fibrotic genes, such as TLR-4 and MMP1 were also higher after LPS stimulation, and their expression was restored after HM sEVs or w3-oxylipins treatment (Figure 4A). To also test the effect of HM sEVs and w3-oxylipins on fibrosis, an in vitro scratch-wound assay was performed on fibroblasts. Stimulation with LPS increased wound closure (37.8 ± 8.4) compared to the control (57.5 ± 4.5) at 24 h. Fibroblasts activated with LPS and further treated with HM sEVs and w3-oxylipins reduced the elevated migration carried out by LPS, reaching control levels (Figure 4B).

### Inflammatory Response to ω3-oxylipins and HM sEVs In Vitro

To observe the effect of HM sEVs or w3-oxylipins on immune system cells, first an *in vitro* pyrogenic assay was performed. 7.5 μg/mL of HM sEVs or 0.5 nM of each of the three ω3-oxylipins were added to PBMCs (Peripheral blood mononuclear cells) to detect whether these treatments could activate the release of pro-inflammatory cytokines such as TNF-α, IL-1β, IL-6 and IL-8. What was observed with respect to the positive control (LPS) is that neither HM sEVs nor w3-oxylipins activated these proinflammatory signaling pathways (Figure 5A). Second, a T-cell activation and

proliferation assay was developed. When w3-oxylipins were added, the proliferation of T-cell was slightly reduced in contrast to the HM sEVs treatment that appeared to slightly stimulate them (Figure 5B). Finally, an *in vitro* macrophage polarization assay to study the ability of HM sEVs or w3-oxylipins to modulate this process was developed. During the differentiation of monocytes to Mφ1, some cultures were treated with HM sEVs or w3-oxylipins, and surface markers were compared with non-treated Mφ1 and Mφ2 differentiated populations by flow cytometry (Figure 5C). Results showed that the percentage of CD14+CD163+ cells, a classic Mφ2 phenotype, was neither modify by HM sEVs nor by w3-oxylipins treatment. However, when the expression of cell surface receptors on differentiated and LPS-stimulated Mφ1 was analyzed, it was observed that treatment with HM sEVs significantly reduced the expression of the co-stimulatory molecules CD80 and CD86, and HLA-DR expression to levels like Mφ2. Treatment with the w3-oxylipins was also able to reduce the expression of these three markers, although to a lesser extent (Figure 5C).

ω3-οxylipins and HM sEVs ameliorate colitis in mouse model

The evident beneficial effects of HM sEVs and ω3-οxylipins *in vitro* motivated us to test their therapeutic potential in an inflammatory bowel disease model. Alterations in the immune response, intestinal necrosis and fibrosis are clinical manifestations of NEC relatively nonspecific that can be easily mixed as other gastrointestinal diseases (É. Tremblay et al., "Gene expression profiling in necrotizing enterocolitis reveals pathways common to those reported in Crohn's disease," BMC Med. Genomics, vol. 9, no. 1, 2016). For this reason, TNBS-induced mouse colitis model of experimental Crohn's disease was used, since it shares common functional alterations with NEC. Balb/c mice were pre-divided into four groups: a healthy sham group, an untreated TNBS group, a treated TNBS group with 50 μg of HM sEVs and a treated TNBS group with 0.5 μg w3-oxylipins. Treatments were dissolved in 50 μL PBS and oral administered by gavage just after induction of acute colitis by TNBS and at 24 and 48 h after. Sham group was gavaged with 50 μL of vehicle (PBS). On the fourth day, mice were sacrificed, and the regenerative and anti-inflammatory effects of the treatments were assessed. The weight loss of the mice was monitored throughout the experiment. The healthy group did not show weight loss, while the TNBS mice group lost almost 20% of their weight. Mice treated with HM sEVs and w3-oxylipins also showed weight loss; however, this lost was stabilized on the third day, reaching a maximum of 10% on the day of sacrifice (Figure 6A). TNBS mice group showed a reduction in colon length compared to healthy mice, while mice treated with HM sEVs and w3-oxylipins showed protection against colon shortening (Figure 6B). Histology of the colon revealed severe mucosal damage in the TNBS group, characterized by fewer intestinal glands, distortion of crypts and profuse inflammatory cell infiltration. In contrast, the TNBS group treated with HM sEVs and w3-oxylipins showed preserved tissue architecture with significantly less histopathological damage (Figure 6C). To further investigate the mechanisms underlying colitis recovery after treatment with HM sEVs and w3-oxylipins, Sirius Red staining to detect tissue collagen fiber content was used. Intestinal fibrosis occurs in the context of inflammation, leading to tissue damage and impaired tissue reconstruction with excessive deposition of extracellular matrix. As expected, it was observed that the percentage of collagen in the colon was significantly higher in the TNBS group than in the healthy group and was significantly lower in the groups treated with HM sEVs or w3-oxylipins (Figure 6D), suggesting that treatment with HM sEVs and w3-oxylipins is able to alleviate intestinal fibrosis in colitis disease.

ω3-oxylipins and HM sEVs promote anti-inflammatory environment in colitis mouse model

To examine the immune response modulation of HM sEVs and *ω*3-oxylipins, we analyzed cytokine expression in the colon tissues. Cytokine protein arrays showed that the levels of several cytokines, including ICAM-1, TIMP-1, CCL2, CXCL9, CXCL13, CXCL1, IL-1$\beta$, TREM-1, IL-1$\alpha$, CXCL11, IL17, and TNF- $\alpha$ were upregulated in the colitis-induced PBS-treated group compared with the normal healthy control group. In contrast, the colitis-induced group treatred with HM sEVs or *ω*3-oxylipins showed greatly reduced levels of these cytokines, some of them being similar to those of the healthy control group. Moreover, anti-inflamatory cytokines like IL-10 or IL-1ra were upregulated in colitis-induced group treatred with HM sEVs or *ω*3-oxylipins (Figure 7A). An imbalance between proinflammatory and anti-inflammatory immune cells and cytokines is one of the key features of colitis that impedes the resolution of inflammation. In this sense, the expression of inflammatory cytokines and macrophage markers in colon tissue was assesed. As shown in Figure 7B, the mRNA expression levels of pro-inflammatory cytokines (TNF-$\alpha$ and IL-6) were significantly lower in HM sEVs and ω3-oxylipins treated groups than in the untreated TNBS group, whereas the opposite was seen for the anti-inflammatory cytokine IL-10. Analysis of Mcp2c-associated genes (Argl, CD206, CCR2 and Cx3CR1) also showed an increase in HM sEVs and w3-oxylipins treated groups. In addition IL-17a and IL-10 in the plasma and colonic tissue were tested by ELISA. The pro-inflammatory cytokine IL-17 was higher in TNBS group, while HM sEVs and w3-oxylipins treatments decreased this upregulation. In contrast, IL-10 was lower in TNBS group and HM sEVs and ω3-οxylipins treatments increased its presence, both in plasma and colon extract (Figure 7C). To gain further insight into how treatments influence macrophage infiltration in the injury site during disease, an immunofluorescence assay was carried out. It was used a classic macrophage marker as F4/F80 combine with CD274 or CD206 to mark Mφ1 or Mφ2, respectively. Results showed

that the ratio $M\varphi1/M\varphi2$ was significantly higher on TNBS untreated group, whereas HM sEVs and w3-oxylipins treatments were able to reverse this ratio, decreasing $M\varphi1$ and increasing $M\varphi2$ presence (Figure 7D). These data indicate that HM sEVs and w3-oxylipins can prevent the inflammatory response in TNBS-induced colitis by regulating macrophage infiltration and cytokine expression.

**Claims**

1. Composition comprising a combination of oxylipins 14-HDHA, 17-HDHA, and 19,20-DiHDPA.

2. Composition according to claim 1 further comprising one or more oxylipins selected from the group consisting of 12,13-DiHOME; 9,10-DiHOME; 14,15-DiHETrE; $PGE_2$; $PGF_{2\alpha}$; 17,18-DiHETE; 11,12-DiHETrE; 9-HOTrE; 20-HETE; 12(S)-HpETE; 12(S)-HEPE; 13-HODE; 5(S)-HEPE; 9-HODE; 15-HETE; 16(17)-EpDPE; 13-HpODE; 13-KODE; 9-HpODE; 15-HpETE; 15-KETE; 11-HETE; 9-KODE; 12-HETE; 8-HETE; 15(S)-HETrE; 9-HETE; 5-HETE; 19(20)-EpDPE; 12(13)-EpOME; 5(S)-HpETE; 9(10)-EpOME; 12-KETE; 5-KETE; 8(9)-EpETrE, and 5(6)-EpETrE.

3. Composition according to claim 1 or 2 wherein the oxylipins are nano or microencapsulated.

4. Use of the composition according to any of claims 1-3 as nutritional supplement.

5. Use of the composition, according to claim 4, wherein the nutritional supplement is added to human milk or infant formula.

6. Use according to claim 5 wherein the nutritional supplement is added to human milk for preterm infants (PIs).

7. Nutritional supplement comprising the composition according to any of claims 1-3.

8. Pharmaceutical composition comprising the composition according to any of claims 1-3.

9. Pharmaceutical composition according to claim 8 further comprising at least one pharmaceutically acceptable carrier and/or excipient.

10. Pharmaceutical composition according to claim 8 or 9 for use in the prevention and/or treatment of intestinal diseases.

11. Pharmaceutical composition according to claim 10 for use in the prevention and/or treatment of intestinal diseases in preterm infants.

12. Pharmaceutical composition according to claim 11 for use in the prevention and/or treatment of necrotising enterocolitis (NEC).

$1 \times 10^{11}$ particles/mL
177nm diameter

**FIG. 1A**

$\zeta = -7{,}7 \pm 1{,}0$ (mV)

**FIG. 1B**

FIG. 1C

FIG. 1D

FIG. 1E

**FIG. 1F**

**FIG. 1G**

**FIG. 2A**

**FIG. 2B**

**FIG. 2C**

EP 4 437 858 A1

FIG. 2D

FIG. 2G

FIG. 2F

FIG. 2E

FIG. 2H

**FIG. 3A**

FIG. 3B

FIG. 3C

FIG. 3D

FIG. 4A

**FIG. 4B**

# Pyrogen test

FIG. 5A

**Immunogen test**

# FIG. 5B

# FIG. 5C

FIG. 6A

FIG. 6B

**FIG. 6C**

FIG. 6D

FIG. 7A

**FIG. 7B**

## Colon extract

## Plasma

# FIG. 7C

FIG. 7D

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/008434 A1 (SERHAN CHARLES NICHOLAS [US] ET AL) 13 January 2022 (2022-01-13) * claims; table 4 * * paragraphs [0129], [0130], [0131], [0134] * * paragraphs [0140], [0141], [0177], [0178] * * paragraphs [0161], [0162], [0163] * | 1-12 | INV. A23L33/12 A23L33/00 A61K31/202 A61P29/00 |
| X | ROBINSON D T ET AL: "Long chain fatty acids and related pro-inflammatory, specialized pro-resolving lipid mediators and their intermediates in preterm human milk during the first month of lactation", PROSTAGLANDINS LEUKOTRIENES AND ESSENTIAL FATTY ACIDS, CHURCHILL LIVINGSTONE, EDINBURGH, vol. 121, 18 May 2017 (2017-05-18), pages 1-6, XP085109101, ISSN: 0952-3278, DOI: 10.1016/J.PLEFA.2017.05.003 * abstract * * table 3 * | 1,2,4,7, 10,11 | |
| A | US 2009/320148 A1 (AATERBURN LINDA MARY [US] ET AL) 24 December 2009 (2009-12-24) * paragraph [0147] * | 12 | TECHNICAL FIELDS SEARCHED (IPC) A23L A61P A61K |

The present search report has been drawn up for all claims

| Place of search | Date of completion of the search | Examiner |
|---|---|---|
| Munich | 23 August 2023 | Vernier, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 38 2313

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2023

| Patent document cited in search report | | Publication date | Patent family member(s) | | Publication date |
|---|---|---|---|---|---|
| US 2022008434 | A1 | 13-01-2022 | US | 2018256597 A1 | 13-09-2018 |
| | | | US | 2020316086 A1 | 08-10-2020 |
| | | | US | 2021038614 A1 | 11-02-2021 |
| | | | US | 2022008434 A1 | 13-01-2022 |
| | | | US | 2023149422 A1 | 18-05-2023 |
| | | | US | 2023149423 A1 | 18-05-2023 |
| | | | WO | 2017041094 A1 | 09-03-2017 |
| US 2009320148 | A1 | 24-12-2009 | EP | 1983977 A2 | 29-10-2008 |
| | | | US | 2009320148 A1 | 24-12-2009 |
| | | | WO | 2007090162 A2 | 09-08-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 202218889 A1

**Non-patent literature cited in the description**

- **ASCANIUS S. R. et al.** Milk-Derived Extracellular Vesicles Suprress Inflammatory Cytorkine Expression and Nuclear Factor-$\kappa$B Activation in Lipopolysaccharide-Stimulated Macrophages. *Dairy,* 2021, vol. 2, 165-178
- **RAMOS-GARCIA et al.** ATR-FTIR spectroscopy for the routine quality control of exosome isolations. *Chemometrics and Intelligent laboratory systems,* 2021
- **THÉRY, C. et al.** Minimal information for studies of extracellular vesicles 2018 (MISEV2018): a position statement of the International Society for Extracellular Vesicles and update of the MISEV2014 guidelines. *J. Extracell. Vesicles,* 2018, vol. 7, 1535750
- **MARTIN, C. R. et al.** Decreased postnatal docosahexaenoic and arachidonic acid blood levels in premature infants are associated with neonatal morbidities. *The Journal of Pediatrics,* 2011, vol. 159 (5), 743-749.e7492
- **CHEN W. et al.** Lipidomic Profiling of Human Milk Derived Exosomes and Their Emerging Roles in the prevention of Necrotizing Enterocolitis. *Mol Nutr. Food Res.,* 2021, 2000845
- **BURATTA S. et al.** Protein and Lipid Content of Milk Extracellular Vesicles: A comparative Overview. *Life,* 2023, vol. 13, 401
- **GILA-DIAZ, A. et al.** Specialized Pro-Resolving Lipid Mediators in Neonatal Cardiovascular Physiology and Diseases. *Antioxidants,* 2021, vol. 10 (6), 933

- **SERHAN CN ; LEVY BD.** Resolvins in inflammation: emergence of the pro-resolving superfamily of mediators. *J Clin Invest.,* 2018, vol. 128 (7), 2657-2669
- **RAMOS-GARCIA, V. et al.** Isolation and Lipidomic Screening of Human Milk Extracellular Vesicles. *Methods in molecular biology (Clifton, N.J.),* 2023, vol. 2571, 177-188
- **GONZALEZ-KING. H. et al.** Hypoxia Inducible Factor-1 a Potentiates Jagged 1-Mediated Angiogenesis by Mesenchymal Stem Cell-Derived Exosomes. *Stem Cells,* 24 April 2017
- **CZERNEK L.** The Uptake of Extracellular Vesicles is Affected by the Differentiation Status of Myeloid Cells. *Immunol,* 2015, vol. 82, 506-514
- **STASSBURG, K. et al.** *Targeted Lipidomics of Oxylipins (Oxygenated Fatty Acids),* 2015
- **A., FERRIN I. et al.** Human mesenchymal stromal cells modulate T-cell responses through TNF-$\alpha$-mediated activation of NF-$\kappa$B. Eur. *J. Immunol.,* 2014, vol. 44, 480-488
- **LYONS A. et al.** Analysing cell division in vivo and in vitro using flow cytometric measurement of CFSE dye dilution. *J. Immunol. Methods.,* 2000, vol. 243, 147-154
- **COSÍN-ROGER J. et al.** The activation of Wnt signaling by a STAT6-dependent macrophage phenotype promotes mucosal repair in murine IBD. *Mucosal Immunol.,* 2015, vol. 9, 986-998
- **É. TREMBLAY et al.** Gene expression profiling in necrotizing enterocolitis reveals pathways common to those reported in Crohn's disease. *BMC Med. Genomics,* 2016, vol. 9 (1